# EUROPEAN PATENT SPECIFICATION

(11) **EP 1 831 369 B1**
(45) Date of publication and mention of the grant of the patent: **19.09.2012**
(21) Application number: 05855060.9
(22) Date of filing: 19.12.2005
(51) Int. Cl.: C12N 15/113, C07K 14/47, A61K 31/713

(54) **RNAI INHIBITION OF SERUM AMYLOID A FOR TREATMENT OF GLAUCOMA**
BEHANDLUNG VON GLAUKOM MITTELS RNAI INHIBIERUNG DES AMYLOIDEN SERUM A
INHIBITION D'ARNI D'AMYLOIDE SERIQUE A POUR LE TRAITEMENT DE GLAUCOME

(30) Priority: 23.12.2004 US 638706 P
(43) Date of publication of application: 12.09.2007
(73) Proprietor: Novartis AG, 4056 Basel (CH)
(72) Inventor: CLARK, Abbot, F., Arlington, Texas 76017 (US); WANG, Wan-Heng, Grapevine, Texas 76051 (US); MCNATT, Loretta, Hurst, Texas 76054 (US)
(74) Representative: Grünecker, Kinkeldey, Stockmair & Schwanhäusser
(86) International application number: PCT/US2005/046436
(87) International publication number: WO 2006/071691

(56) References cited:
- WO-A-01/64949
- WO-A-03/012057
- WO-A-2004/022782
- WO-A-2005/060542
- WO-A-2005/079815
- URIELI-SHOVAL S ET AL: "Widespread expression of serum amyloid A in histologically normal human tissues: Predominant localization to the epithelium" JOURNAL OF HISTOCHEMISTRY AND CYTOCHEMISTRY, HISTOCHEMICAL SOCIETY, NEW YORK, NY, US, vol. 46, no. 12, December 1998 (1998-12), pages 1377-1384, XP002298394 ISSN: 0022-1554
- ROZSA FRANK W ET AL: "Gene expression profile of human trabecular meshwork cells in response to long-term dexamethasone exposure." MOLECULAR VISION [ELECTRONIC RESOURCE]. 2006, vol. 12, 2006, pages 125-141, XP002386049 ISSN: 1090-0535

## Description

### FIELD OF THE INVENTION

The present invention relates to the field of interfering RNA compositions for inhibition of expression of serum amyloid A (SAA) in glaucoma, particularly for primary open angle glaucoma.

### BACKGROUND OF THE INVENTION

Glaucoma is a heterogeneous group of optic neuropathies that share certain clinical features. The loss of vision in glaucoma is due to the selective death of retinal ganglion cells in the neural retina that is clinically diagnosed by characteristic changes in the visual field, nerve fiber layer defects, and a progressive cupping of the optic nerve head (ONH). One of the main risk factors for the development of glaucoma is the presence of ocular hypertension (elevated intraocular pressure, IOP). An adequate intraocular pressure is needed to maintain the shape of the eye and to provide a pressure gradient to allow for the flow of aqueous humor to the avascular cornea and lens. IOP also appears to be involved in the pathogenesis of normal tension glaucoma where patients have what is often considered to be normal IOP.

The elevated IOP associated with glaucoma is due to elevated aqueous humor outflow resistance in the trabecular meshwork (TM), a small specialized tissue located in the iris-corneal angle of the ocular anterior chamber. Glaucomatous changes to the TM include a loss in TM cells and the deposition and accumulation of extracellular debris including proteinaceous plaque-like material. In addition, there are also changes that occur in the glaucomatous ONH. In glaucomatous eyes, there are morphological and mobility changes in ONH glial cells. In response to elevated IOP and/or transient ischemic insults, there is a change in the composition of the ONH extracellular matrix and alterations in the glial cell and retinal ganglion cell axon morphologies.

Primary glaucomas result from disturbances in the flow of intraocular fluid that has an anatomical or physiological basis. Secondary glaucomas occur as a result of injury or trauma to the eye or a preexisting disease. Primary open angle glaucoma (POAG), also known as chronic or simple glaucoma, represents ninety percent of all primary glaucomas. POAG is characterized by the degeneration of the trabecular meshwork, resulting in abnormally high resistance to fluid drainage from the eye. A consequence of such resistance is an increase in the IOP that is required to drive the fluid normally produced by the eye across the increased resistance.

Current anti-glaucoma therapies include lowering IOP by the use of suppressants of aqueous humor formation or agents that enhance uveoscleral outflow, laser trabeculoplasty, or trabeculectomy which is a filtration surgery to improve drainage. Pharmaceutical anti-glaucoma approaches have exhibited various undesirable side effects. For example, miotics such as pilocarpine can cause blurring of vision and other negative visual side effects. Systemically administered carbonic anhydrase inhibitors can also cause nausea, dyspepsia, fatigue, and metabolic acidosis. Further, certain beta-blockers have increasingly become associated with serious pulmonary side effects attributable to their effects on beta-2 receptors in pulmonary tissue. Sympathomimetics cause tachycardia, arrhythmia and hypertension. Such negative side effects may lead to decreased patient compliance or to termination of therapy.

More importantly, the current anti-glaucoma therapies do not directly address the pathological damage to the trabecular meshwork, the optic nerve, and loss of retinal ganglion cells and axons, which continues unabated. In view of the importance of glaucoma, and the inadequacies of prior methods of treatment, it would be desirable to have an improved method of treating glaucoma that would address the underlying causes of its progression.

### SUMMARY OF THE INVENTION

The present invention is directed to interfering RNAs that target SAA mRNA and thereby interfere with SAA mRNA expression. The interfering RNAs of the invention are useful for treating SAA-related glaucoma. The present invention is defined by the following embodiments 1 to 16
1. A composition comprising an effective amount of interfering RNA having a length of 19 to 49 nucleotides and a pharmaceutically acceptable carrier, the interfering RNA comprising: a sense nucleotide sequence, an antisense nucleotide sequence, and a region of at least 80% contiguous complementarity of at least 19 nucleotides between the sense and antisense sequences; wherein the antisense sequence hybridizes under physiological conditions to a portion of mRNA corresponding to SEQ ID NO: 1 or SEQ ID NO: 2 , and has a region of at least 80% contiguous complementarity of at least 19 nucleotides with the hybridizing portion of mRNA corresponding to SEQ ID NO: 1 or SEQ ID NO: 2 for use in treating serum amyloid A-associated glaucoma in an eye of a subject.
2. A composition comprising an effective amount of interfering RNA having a length of 19 to 49 nucleotides and a pharmaceutically acceptable carrier, the interfering RNA comprising: a nucleotide sequence having a region of at least 80% contiguous complementarity of at least 19 nucleotides with a hybridizing portion of mRNA corresponding to SEQ ID NO: 1 or SEQ ID NO: 2, wherein the nucleotide sequence hybridizes under physiological conditions to a portion of mRNA corresponding to SEQ ID NO: 1 or SEQ ID NO: 2, for use in treating amyloid A-associated glaucoma in an eye of a subject.
3. An *in vitro* method for attenuating expression of serum amyloid A mRNA comprising administering an effective amount of interfering RNA having a length of 19 to 49 nucleotides and a pharmaceutically acceptable carrier, the interfering RNA comprising: a sense nucleotide sequence, an antisense nucleotide sequence, and a region of at least 80% contiguous complementarity of at least 19 nucleotides between the sense and antisense sequences; wherein the antisense sequence hybridizes under physiological conditions to a portion of mRNA corresponding to SEQ ID NO: 1 or SEQ ID NO:2, and has a region of at least 80% contiguous complementarity of at least 19 nucleotides with the hybridizing portion of mRNA corresponding to SEQ ID NO: 1 or SEQ ID NO:2 to a cell.
4. An *in vitro* method for attenuating expression of serum amyloid A mRNA comprising administering an effective amount of interfering RNA having a length of 19 to 49 nucleotides and a pharmaceutically acceptable carrier, the interfering RNA comprising: a nucleotide sequence having a region of at least 80% contiguous complementarity of at least 19 nucleotides with a hybridizing portion of mRNA corresponding to SEQ ID NO: 1 or SEQ ID NO:2, wherein the nucleotide sequence hybridizes under physiological conditions to a portion of mRNA corresponding to SEQ ID NO: 1 or SEQ ID NO: 2 to a cell.
5. The composition or method of item 1 or 3, wherein the antisense sequence has a region of at least 80% contiguous complementarity of at least 21 to 23 nucleotides with the hybridizing portion of mRNA corresponding to SEQ ID NO: 1 or SEQ ID NO: 2, and comprises an additional TT sequence at the 3' end of each of the sense and the antisense sequence.
6. The composition or method of item 1, 3 or 5, wherein the sense nucleotide sequence and the antisense nucleotide sequence are connected by a loop nucleotide sequence.
7. The composition or method of any of items 1, 3, 5 and 6 wherein the antisense sequence is designed to target a nucleotide sequence of mRNA corresponding to SEQ ID NO: 1 beginning at nucleotide 230, 357, 362, 380, 447, 470, 527, 531, 548, or 557.
8. The composition or method of any of items 1, 3, 5 and 6 wherein the antisense sequence is designed to target a nucleotide sequence of mRNA corresponding to SEQ ID NO: 2 beginning at nucleotide 43, 170, 175, 193, 260, 283, 339, or 370.
9. The composition or method of any of items 1, 3, 5, 6 and 8 wherein the antisense sequence is designed to target a nucleotide sequence of mRNA corresponding to SEQ ID NO:2 beginning at nucleotide 252, 271, 276, 325, 343.
10. The composition or method of any of items 1, 3, 5 to 9 wherein the antisense sequence comprises
   CUUUGCCACUCCUGCCCCA (SEQ ID NO:37), UCGGAAGUGAUUGGGGUCU (SEQ ID NO:38),
   UUUGUCUGAGCCGAUGUAA (SEQ ID NO:39), AACCAGGCCCGUGAGAAGC (SEQ ID NO:40),
   CUGAGCCGAUGUAAUUGGC (SEQ ID NO:41), GCCACUCCUGCCCCAUUUA (SEQ ID NO:69),
   CCCCCGAGCAUGGAAGUAU (SEQ ID NO:42), CUCUGGCAUUGCUGAUCAC (SEQ ID NO:43),
   GCCUGUGAGUCUCUGGAUA (SEQ ID NO:44), GCCACUCCUGCCCCAUUUA (SEQ ID NO:45),
   GCCAGCAGGUCGGAAGUGA (SEQ ID NO: 46), AGUCUCUGGAUAUUCUCUC(SEQ ID NO:47),
   UUUAUUGGCAGCCUGAUCG(SEQIDNO:48), UUGCUGAUCACUUCUGCGG(SEQIDNO:49),
   CUGGAUAUUCUCUCUGGCA(SEQIDNO:50), UCUGCCACUCCUGCCCCAU(SEQIDNO: 51),
   AACCCCUUGGAGAGCCUCC(SEQIDNO:52), UGCCCAUGUCCCCAACCCC(SEQIDNO:53),
   AUAGAGAUAUCUGUUUGAA(SEQIDNO:54), CGAGCAUAGAGAUAUCUGU(SEQIDNO:55),
   CUUUGGGCAGCAUCAUAGU(SEQIDNO:56), AGACACCCCCAGGUCCUCU(SEQIDNO:57),
   CCUGGAACGGCUGAUGAGU(SEQIDNO:58), CCAAAUAAAUAGUAGUCUA(SEQIDNO:59),
   UCCAAUACAGUGCUGCUGU(SEQIDNO:60), CUCAGCUUUCUCGUUGGAC(SEQIDNO:61),
   CCAUUCCUCAGCUUUCUCG(SEQIDNO:62), CCGGCCCCAUUCCUCAGCU(SEQIDNO:63),
   CUUUGCCACUCCGGCCCCA(SEQIDNO:64),or UCUGAAGCGGUCGGGGUCU(SEQIDNO:65).
11. The composition or method of any preceding item wherein the interfering RNA comprises a modification on a base portion, on a sugar portion or on a phosphate portion.
12. The composition or method of any of items 1, 3 and 5, wherein the composition further comprises a second interfering RNA having a length of 19 to 49 nucleotides, and comprising a sense nucleotide sequence, an antisense nucleotide sequence, and a region of at least 80% complementarity of at least 19 nucleotides between the sense and antisense sequences; wherein the antisense sequence of the second interfering RNA hybridizes under physiological conditions to a second portion of mRNA corresponding to SEQ ID NO: 1 or SEQ ID NO: 2 and the antisense sequence has a region of at least 80% contiguous complementarity of at least 19 nucleotides with the second hybridizing portion of mRNA corresponding to SEQ ID NO: 1 or SEQ ID NO: 2.
13. The composition of any of items 1, 3 and 5, wherein the composition comprises an effective amount of a mixture of at least four interfering RNAs, each interfering RNA having a length of 19 to 49 nucleotides, and a pharmaceutically acceptable carrier, each interfering RNA comprising: a sense nucleotide sequence, an antisense nucleotide sequence, and a region of at least 80% contiguous complementarity of at least 19 nucleotides between the sense and antisense sequences of each of the four interfering RNAs; wherein the antisense sequences of the mixture hybridize under physiological conditions to a portion of mRNA corresponding to SEQ ID NO: 2 beginning at nucleotide 175, 252, 276, and 325, respectively, and have a region of at least 80% contiguous complementarity of at least 19 nucleotides with the hybridizing portion of mRNA corresponding to SEQ ID NO: 2 beginning at nucleotide 175, 252, 276, and 325, respectively.
14. The composition of item 2 or the method of item 4 wherein the composition further comprises a second interfering RNA having a length of 19 to 49 nucleotides, and comprising a second nucleotide sequence having a region of at least 80% contiguous complementarity of at least 19 nucleotides with a second hybridizing portion of mRNA corresponding to SEQ ID NO: 1 or SEQ ID NO: 2.
15. The composition of item 2 or the method of item 4 wherein the composition comprises an effective amount of a mixture of at least four interfering RNAs, each interfering RNA having a length of 19 to 49 nucleotides, and the mixture comprising: a first, second, third and fourth nucleotide sequence having a region of at least 80 % contiguous complementarity of at least 19 nucleotides with the hybridizing portion of mRNA corresponding to SEQ ID NO: 2 beginning at nucleotide 175, 252, 276, and 325, respectively.
16. The composition or method of any preceding item wherein the composition is prepared for administration via a topical, intravitreal, or transcleral route.

Disclosed herein is a method of attenuating expression of serum amyloid A mRNA in an eye of a subject. The method comprises administering to the eye of the subject a composition comprising an effective amount of interfering RNA such as double-stranded (ds) siRNA or single-stranded (ss) siRNA having a length of 19 to 49 nucleotides and a pharmaceutically acceptable carrier.

The double stranded siRNA comprises a sense nucleotide sequence, an antisense nucleotide sequence and a region of at least near-perfect contiguous complementarity of at least 19 nucleotides. Further, the antisense sequence hybridizes under physiological conditions to a portion of mRNA corresponding to SEQ ID NO:1, SEQ ID NO:2, or SEQ ID NO:3 which are sense sequences of DNA that encode SAA1, SAA2, and SAA4, respectively (GenBank reference no. NM_000331, BC020795, and NM_006512) and has a region of at least near-perfect contiguous complementarity of at least 19 nucleotides with the hybridizing portion of mRNA corresponding to SEQ ID NO:1, SEQ ID NO:2, or SEQ ID NO:3, respectively. The administration of such a composition attenuates the expression of serum amyloid A mRNA of the eye of the subject.

When the interfering RNA is single-stranded, the interfering RNA comprises a nucleotide sequence having a region of at least near-perfect contiguous complementarity of at least 19 nucleotides with a hybridizing portion of mRNA corresponding to SEQ ID NO:1, SEQ ID NO:2, or SEQ ID NO:3.

In one embodiment of the invention, antisense siRNA is designed to target a nucleotide sequence of mRNA corresponding to SEQ ID NO:1 beginning at nucleotide 230, 357, 362, 380, 447, 470, 527, 531, 548, or 557. In another embodiment of the invention, the antisense sequence is designed to target a nucleotide sequence of mRNA corresponding to SEQ ID NO:2 beginning at nucleotide 43, 170, 175, 193, 260, 283, 339, or 370. In a further embodiment of the invention, the antisense sequence is designed to target a nucleotide sequence of mRNA corresponding to SEQ ID NO:2 beginning at nucleotide 252, 271, 276, 325, or 343. In yet a further embodiment of the invention, the antisense sequence is designed to target a nucleotide sequence of mRNA corresponding to SEQ ID NO:3 beginning at nucleotide 153, 166, 222, 227, 251, 268, 297, 335, 356, 384, 390, 396, 406, or 423.

Further disclosed herein is a method of treating a serum amyloid A-associated glaucoma in a subject in need thereof. The method comprises administering to the eye of the subject a composition comprising an effective amount of interfering RNA having a length of 19 to 49 nucleotides and a pharmaceutically acceptable carrier, the interfering RNA comprising a sense nucleotide sequence, an antisense nucleotide sequence, and a region of at least near-perfect contiguous complementarity of at least 19 nucleotides. The antisense sequence hybridizes under physiological conditions to a portion of mRNA corresponding to SEQ ID NO:1, SEQ ID NO; 2, or SEQ ID NO:3, and has a region of at least near-perfect contiguous complementarity of at least 19 nucleotides with the hybridizing portion of mRNA corresponding to SEQ ID NO:1, SEQ ID NO:2, or SEQ ID NO:3, respectively. The serum amyloid A-associated glaucoma is treated thereby.

### BRIEF DESCRIPTION OF THE DRAWINGS

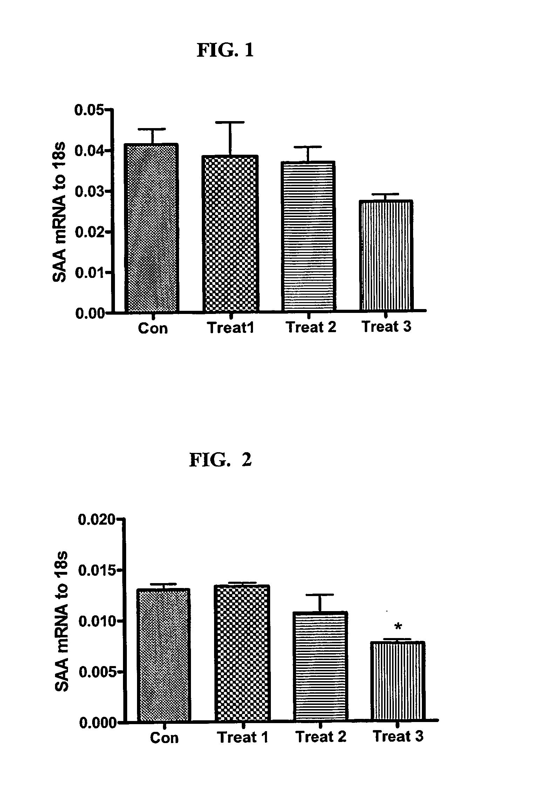

FIG. 1 provides a QPCR analysis of SAA2 mRNA/18s rRNA ratio to examine the effect of siRNA on endogenous SAA mRNA in NTM 765 normal trabecular meshwork cells transfected with SMARTPOOL^{®} siRNA targeting SAA mRNA. Trabecular meshwork cells were transfected with 100 nM of the siRNA using Dharmafect #1 reagent at three different concentrations for 24 hrs: Con: Control; Treat 1: Treatment 1 at 0.05 µl/100µl well; Treat 2: Treatment 2 at 0.2 µl/100µl well; Treat 3: Treatment 3 at 0.4 µl/100µl well.

FIG. 2 provides a QPCR analysis of SAA2 mRNA/18s rRNA ratio to examine the effect of siRNA on endogenous SAA mRNA in GTM686 glaucomatous trabecular meshwork cells transfected with SMARTPOOL^{®} siRNA targeting SAA mRNA. Trabecular meshwork cells were transfected with 100 nM of the siRNA using Dharmafect #1 reagent at three different concentrations for 24 hrs: Con: Control; Treat 1: Treatment 1 at 0.05 µl/100µl well; Treat 2: Treatment 2 at 0.2 µl/100µl well; Treat 3: Treatment 3 at 0.4 µl/100µl well. *: p<0.05 vs. both control and Treatment 1 by one-way ANOVA then Newman-Keuls Multiple Comparison Test.

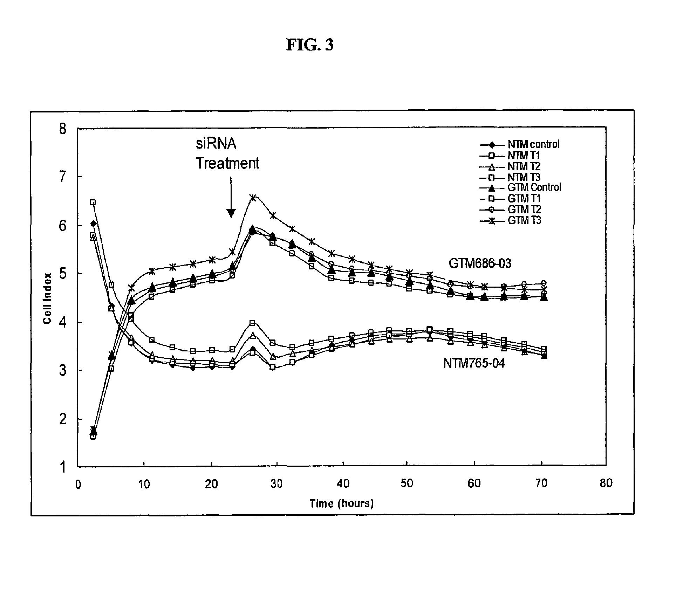

FIG. 3 provides real time electronic monitoring (RE-CES™) of the effect of SAA siRNA treatment on the growth and morphology of normal (NTM 765-04) and glaucomatous (GTM 686-03) trabecular meshwork cells. The cells were transfected with 100 nM of SMARTPOOL^{®} siRNA targeting SAA mRNA using Dharmafect #1 reagent at three different concentrations for 48 hrs: T1: 0.05 µl/100µl well; T2: 0.2 µl/100µl well; T3: 0.4 µl/100µl well.

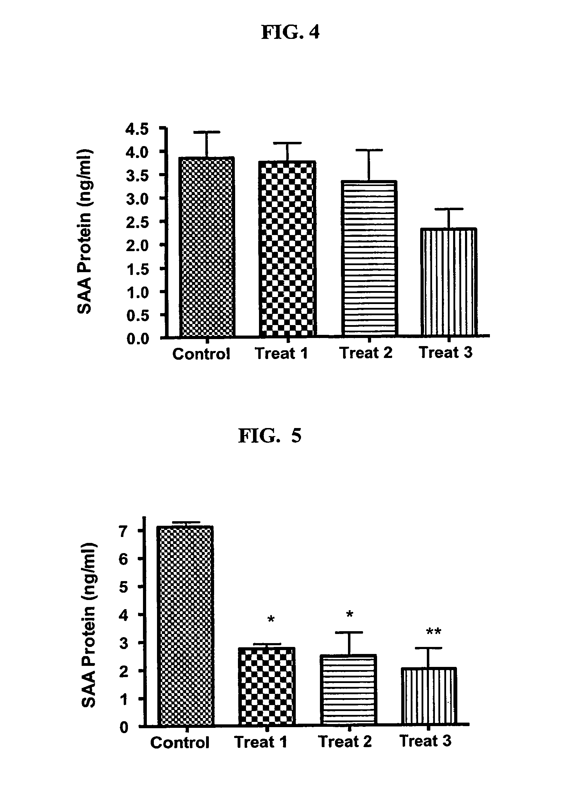

FIG. 4 provides results of an ELISA assay for the level of endogenous SAA protein in siRNA-treated NTM765 normal trabecular meshwork cell lysates. The cells were transfected with 100 nM of SAA SMARTPOOL^{®} siRNA targeting SAA mRNA using Dharmafect #1 reagent at three different concentrations for 48 hr: Treat 1: Treatment 1 at 0.05 µl/100µl well; Treat 2: Treatment 2 at 0.2 µl/100µl well; Treat 3: Treatment 3 at 0.4 µl/100µl well.

FIG. 5 provides results of an ELISA assay for the level of endogenous SAA protein in siRNA-treated GTM686 glaucomatous trabecular meshwork cell lysates. The cells were transfected with 100nM of SAA SMARTPOOL^{®} siRNA targeting SAA mRNA using Dharmafect #1 reagent at three different concentrations for 48 hr: Treat 1: Treatment 1 at 0.05 µl/100µl well; Treat 2: Treatment 2 at 0.2 µl/100µl well; Treat 3: Treatment 3 at 0.4 µl/100µl well. * : p<0.05; **: p<0.01 vs. control by ANOVA then Bonferroni's Multiple Comparison Test.

### DETAILED DESCRIPTION OF THE INVENTION

RNA interference, termed "RNAi," is a method for reducing the expression of a target gene that is effected by small single- or double-stranded RNA molecules. Interfering RNAs include small interfering RNAs, either double-stranded or single-stranded (ds siRNAs or ss siRNAs), microRNAs (miRNAs), small hairpin RNAs (shRNAs), and others. While not wanting to be bound by theory, RNA interference appears to occur *in vivo* with the cleavage of dsRNA precursors into small RNAs of about 20 to 25 nucleotides in length. Cleavage is accomplished by RNaseIII-RNA helicase Dicer. The "sense" strand of an siRNA, i.e., the strand that has exactly the same sequence as a target mRNA sequence, is removed, leaving the 'antisense" strand which is complementary to the target mRNA to function in reducing expression of the mRNA. The antisense strand of the siRNA appears to guide a protein complex known as RISC (RNA-induced silencing complex) to the mRNA, which complex then cleaves the mRNA by the Argonaute protein of the RISC, thereby reducing protein production by that mRNA. Interfering RNAs are catalytic and reduction in expression of mRNA can be achieved with substoichiometric amounts of interfering RNAs in relation to mRNA. Reduction in mRNA expression may also occur via transcriptional and translational mechanisms.

The present invention relates to the use of interfering RNA for inhibition of expression of serum amyloid A (SAA) in ocular disorders. According to the present invention, exogenously provided siRNAs effect silencing of SAA mRNA of ocular structures. The present inventors have previously shown that the expression of serum amyloid A (SAA) mRNA and protein are significantly upregulated in glaucomatous TM tissues and cells (pending U.S. patent application USSN 60/530,430, entitled "Use of Serum Amyloid A Gene in Diagnosis and Treatment of Glaucoma and Identification of Anti-Glaucoma Agents" filed December 17, 2003. The present inventors have verified the differential mRNA expression seen using Affymetrix gene chips by real time quantitative polymerase chain reaction (QPCR) and increased SAA protein levels by SAA ELISA (pending U.S. patent application cited above ).

Nucleic acid sequences cited herein are written in a 5' to 3' direction unless indicated otherwise. The term "nucleic acid," as used herein, refers to either DNA or RNA or a modified form thereof comprising the purine or pyrimidine bases present in DNA (adenine "A," cytosine "C," guanine "G," thymine "T") or in RNA (adenine "A," cytosine "C," guanine "G," uracil "U"). Interfering RNAs provided herein may comprise "T" bases, particularly at 3' ends, even though "T" bases do not naturally occur in RNA. "Nucleic acid" includes the terms "oligonucleotide" and "polynucleotide" and can refer to a single stranded molecule or a double stranded molecule. A double stranded molecule is formed by Watson-Crick base pairing between A and T bases, C and G bases, and A and U bases. The strands of a double stranded molecule may have partial, substantial or full complementarity to each other and will form a duplex hybrid, the strength of bonding of which is dependent upon the nature and degree of complementarity of the sequence of bases. A mRNA sequence is readily determined by knowing the sense or antisense strand sequence of DNA encoding therefor. For example, SEQ ID NO:1 provides the sense strand sequence of DNA corresponding to the mRNA for serum amyloid A1. The sequence of mRNA is identical to the sequence of the sense strand of DNA with the "T" bases replaced with "U" residues. Therefore, the mRNA sequence of serum amyloid A1 is known from SEQ ID NO:1, the mRNA sequence of serum amyloid A2 is known from SEQ ID NO:2, and the mRNA sequence of serum amyloid A4 is known from SEQ ID NO:3.

*Serum Amyloid A mRNA:* Human serum amyloid A comprises a number of small, differentially expressed apolipoproteins encoded by genes localized on the short arm of chromosome 11. There are four isoforms of SAAs. The GenBank database of the National Center for Biotechnology Information at ncbi.nlm.nih.gov provides the corresponding DNA sequence for the messenger RNA of serum amyloid A1 as reference no. NM_000331, provided below as SEQ ID NO:1. The coding sequence for serum amyloid A1 is from nucleotides 225-593.
SAA1: SEQ ID NO:1:

Equivalents of the above cited SAA1 mRNA sequence are alternative splice forms, allelic forms, or a cognate thereof. A cognate is a serum amyloid A1 mRNA from another mammalian species that is homologous to SEQ ID NO:1. SAA1 nucleic acid sequences related to SEQ ID NO:1 are those having GenBank accession numbers NM_009117 (from mouse), NM_199161 (a human transcript variant 2), BC007022.1, BG533276.1, BG567902.1, BQ691948.1, CD102084.1, M10906.1, M23698.1, X51439.1, X51441.1, X51442.1, X51443.1 and X56652.1.

The GenBank database provides the corresponding DNA sequence for the messenger RNA of serum amyloid A2 as reference no. NM_BC020795, provided below as SEQ ID NO:2. The coding sequence for serum amyloid A2 is from nucleotides 38-406.
SAA2: SEQ ID NO:2

Equivalents of the above cited SAA2 mRNA sequence are alternative splice forms, allelic forms, or a cognate thereof. A cognate is a serum amyloid A2 mRNA from another mammalian species that is homologous to SEQ ID NO:2. SAA2 nucleic acid sequences related to SEQ ID NO:2 are those having GenBank accession numbers NM_030754 (human) BC058008.1, J03474.1, L05921.1, M23699.1, M23700.1, M26152.1, X51440.1, X51444.1, X51445.1, and X56653.1.

The proteins products of SEQ ID NO:1 and SEQ ID NO:2 (SAA1 and SAA2) are known as acute phase reactants and similar to C-reactive protein, they are dramatically upregulated by proinflammatory cytokines. SAA1 and SAA2 proteins are 93.5% identical at the amino acid level and the genes are 96.7% identical at the nucleotide level.

The GenBank database provides the corresponding DNA sequence for the messenger RNA of serum amyloid A4 as reference no. NM_006512, provided below as SEQ ID NO:3. The coding sequence for serum amyloid A4 is from nucleotides 76 - 468.
SAA4: SEQ ID NO.3

SAA4 is a low level constitutively expressed gene. Equivalents of the above cited SAA4 mRNA sequence are alternative splice forms, allelic forms, or a cognate thereof. A cognate is a serum amyloid A4 mRNA from another mammalian species that is homologous to SEQ ID NO:3. SAA4 nucleic acid sequences related to SEQ ID NO:3 are those having GenBank accession numbers BC007026, M81349.1, and S48983.1.

*Attenuating expression of an mRNA:* The phrase, "attenuating expression of an mRNA," as used herein, means administering an amount of interfering RNA to effect a reduction of the full mRNA transcript levels of a target gene in a cell, thereby decreasing translation of the mRNA into protein as compared to a control RNA having a scrambled sequence. The reduction in expression of the mRNA is commonly referred to as "knock-down" of mRNA. Knock-down of expression of an amount including and between 50% and 100% is contemplated by embodiments herein. However, it is not necessary that such knock-down levels be achieved for purposes of the present invention. Further, two sets of interfering RNAs may be mildly effective at knock-down individually, however, when administered together may be significantly more effective. In one embodiment, an individual ds siRNA is effective at knock-down at up to 70%. In another embodiment, two or more ds si RNAs are together effective at knock-down at up to 70%.

Knock-down is commonly measured by determining the mRNA levels by Quantitative Polymerase Chain Reaction (QPCR) amplification or by determining protein levels by Western Blot or enzyme linked immunosorbent assay (ELISA). Analyzing the protein level provides an assessment of both mRNA degradation by the RNA Induced Silencing Complex (RISC) as well as translation inhibition. Further techniques for measuring knock-down include RNA solution hybridization, nuclease protection, Northern hybridization, reverse transcription, gene expression monitoring with a microarray, antibody binding, radioimmunoassay, and fluorescence activated cell analysis.

Inhibition of SAA is also inferred in a human or mammal by observing an improvement in a glaucoma symptom such as improvement in intraocular pressure, improvement in visual field loss, or improvement in optic nerve head changes, for example.

Interfering RNA of embodiments of the invention act in a catalytic manner, i.e., interfering RNA is able to effect inhibition of target mRNA in substoichiometric amounts. As compared to antisense therapies, significantly less interfering RNA is required to provide a therapeutic effect.

*Double-stranded interfering RNA:* Double stranded interfering RNA (also referred to as ds siRNA), as used herein, has a sense nucleotide sequence and an antisense nucleotide sequence, the sense and antisense sequence comprising a region of at least near-perfect contiguous complementarity of at least 19 nucleotides. The length of the interfering RNA comprises 19 to 49 nucleotides, and may comprise a length of 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or 49 nucleotides. The antisense sequence of the ds siRNA hybridizes under physiological conditions to a portion of SEQ ID NO:1, SEQ ID NO:2, or SEQ ID NO:3 and has a region of at least near-perfect contiguous complementarity of at least 19 nucleotides with the hybridizing portion of SEQ ID NO:1, SEQ ID NO:2, or SEQ ID NO:3, respectively.

The antisense strand of the siRNA is the active guiding agent of the siRNA in that the antisense strand binds to a RISC complex within a cell, and guides the bound complex to bind with specificity to the mRNA at a sequence complementary to the sequence of the antisense RNA, thereby allowing subsequent cleavage of the mRNA by the bound complex.

Techniques for selecting target sequences for siRNAs are provided by Tuschl, T. et al., "The siRNA User Guide," revised May 6, 2004, available on the Rockefeller University web site, by Technical Bulletin #506, "siRNA Design Guidelines," Ambion Inc. at Ambion's web site, by the Invitrogen web site using search parameters of min 35%, max 55% G/C content, and by the Dharmacon web site. The target sequence may be located in the coding region or a 5' or 3' untranslated region of the mRNA.

An embodiment of a DNA target sequence for SAA1 is present at nucleotides 531 to 549 of SEQ ID NO:1:
5'-TGGGGCAGGAGTGGCAAAG -3' SEQ ID NO:4.
A double stranded siRNA of the invention for targeting a corresponding mRNA sequence of SEQ ID NO:4 and having a 3'UU overhang on each strand is:
5'-UGGGGCAGGAGUGGCAAAGUU-3' SEQ ID NO:5
3' -UUACCCCGUCCUCACCGUUUC-5' SEQ ID NO:6.
The 3' overhang may have a number of "U" residues, for example, a number of "U" residues between and including 2, 3, 4, 5, and 6. The 5' end may also have a 5' overhang of nucleotides. A double stranded siRNA of the invention for targeting a corresponding mRNA sequence of SEQ ID NO:4 and having a 3'TT overhang on each strand is:
5'-UGGGGCAGGAGUGGCAAAGTT-3' SEQ ID NO:7
3' - TTACCCCGUCCUCACCGUUUC-5' SEQ ID NO:8.
The strands of a double-stranded siRNA may be connected by a hairpin loop to form a single stranded siRNA as follows: N is a nucleotide A, T, C, G, U, or a modified form known by one of ordinary skill in the art. The number of nucleotides N is a number between and including 3 to 23, or 5 to 15, or 7 to 13, or 4 to 9, or 9 to 11, or the number of nucleotides N is 9.

Table 1 lists examples of SAA DNA target sequences of SEQ ID NO:1, SEQ ID NO:2, and SEQ ID NO:3 from which siRNAs of the present invention are designed in a manner as set forth above.

**Table 1. SAA Target Sequences for siRNAs**

| **SAA1 Target Sequence** | **# of Starting Nucleotide with reference to SEQ ID NO:1** | **SEQ ID NO:** |
|---|---|---|
| TGGGGCAGGAGTGGCAAAG | 531 | 4 |
| AGACCCCAATCACTTCCGA | 548 | 10 |
| TATCCAGAGATTCTTTGGC | 470 | 66 |
| TGAATGGGGCAGGAGTGGC | 527 | 67 |
| | | |

| **SAA1 and SAA2 Target Sequence in common** | **# of Starting Nucleotide with reference to SEQ ID NO:1 (and with reference to SEQ ID NO:2 in parentheses)** | **SEQ ID NO:** |
|---|---|---|
| TTACATCGGCTCAGACAAA | 362 (175) | 11 |
| GCTTCTCACGGGCCTGGTT | 230 (43) | 12 |
| GCCAATTACATCGGCTCAG | 357 (170) | 13 |
| ATACTTCCATGCTCGGGGG | 380 (193) | 14 |
| GTGATCAGCAATGCCAGAG | 447 (260) | 15 |
| TATCCAGAGACTCACAGGC | 470 (283) | 16 |
| TCACTTCCGACCTGCTGGC | 557 (370) | 17 |
| | | |

| **SAA2 Target Sequence** | **# of Starting Nucleotide with reference to SEQ ID NO:2** | **SEQ ID NO:** |
|---|---|---|
| GAGAGAATATCCAGAGACT | 276 | 18 |
| CGATCAGGCTGCCAATAAA | 325 | 19 |
| CCGCAGAAGTGATCAGCAA | 252 | 20 |
| TGCCAGAGAGAATATCCAG | 271 | 21 |
| ATGGGGCAGGAGTGGCAGA | 343 | 22 |
| TAAATGGGGCAGGAGTGGC | 340 | 68 |
| | | |

| **SAA4 Target Sequence** | **# of Starting Nucleotide with reference to SEQ ID NO:3** | **SEQ ID NO:** |
|---|---|---|
| GGAGGCTCTCCAAGGGGTT | 153 | 23 |
| GGGGTTGGGGACATGGGCA | 166 | 24 |
| TTCAAACAGATATCTCTAT | 222 | 25 |
| ACAGATATCTCTATGCTCG | 227 | 26 |
| ACTATGATGCTGCCCAAAG | 251 | 27 |
| AGAGGACCTGGGGGTGTCT | 268 | 28 |
| ACTCATCAGCCGTTCCAGG | 297 | 29 |
| TAGACTACTATTTATTTGG | 335 | 30 |
| ACAGCAGCACTGTATTGGA | 356 | 31 |
| GTCCAACGAGAAAGCTGAG | 384 | 32 |
| CGAGAAAGCTGAGGAATGG | 390 | 33 |
| AGCTGAGGAATGGGGCCGG | 396 | 34 |
| TGGGGCCGGAGTGGCAAAG | 406 | 35 |
| AGACCCCGACCGCTTCAGA | 423 | 36 |

As cited in the examples above, one of skill in the art is able to use the target sequence information provided in Table 1 to design interfering RNAs having a length shorter or longer than the sequences provided in Table 1 by referring to the sequence position in SEQ ID NO:1, SEQ ID NO:2, or SEQ ID NO:3 and adding or deleting nucleotides complementary or near complementary to SEQ ID NO:1, SEQ ID NO:2, or SEQ ID NO:3, respectively.

The target RNA cleavage reaction guided by ds or ss siRNAs is highly sequence specific. In general, siRNA containing a sense nucleotide sequence identical to a portion of the target mRNA and an antisense portion exactly complementary to the mRNA sense sequence are siRNA embodiments for inhibition of SAA mRNA. However, 100% sequence complementarity between the antisense strand of siRNA and the target mRNA is not required to practice the present invention. Thus the invention allows for sequence variations that might be expected due to genetic mutation, strain polymorphism, or evolutionary divergence. For example, siRNA sequences with insertions, deletions, or single point mutations relative to the target sequence are effective for inhibition.

In certain embodiments of the invention, the antisense sequence comprises CUUUGCCACUCCUGCCCCA (SEQ ID NO:37) or UCGGAAGUGAUUGGGGUCU (SEQ ID NO:38) and the antisense sequence hybridizes to a portion of mRNA corresponding to SEQ ID NO:1.

In further embodiments of the invention, the antisense sequence comprises UUUGUCUGAGCCGAUGUAA (SEQ ID NO:39), AACCAGGCCCGUGAGAAGC (SEQ ID NO:40), CUGAGCCGAUGUAAUUGGC (SEQ ID NO:41), CCCCCGAGCAUGGAAGUAU (SEQ ID NO:42), CUCUGGCAUUGCUGAUCAC (SEQ ID NO:43), GCCUGUGAGUCUCUGGAUA (SEQ ID NO:44), GCCACUCCUGCCCCAUUUA (SEQ ID NO:45), GCCAGCAGGUCGGAAGUGA (SEQ ID NO:46), and the antisense sequence hybridizes to a portion of mRNA corresponding to SEQ ID NO:1 or a portion of mRNA corresponding to SEQ ID NO:2.

In another embodiment of the invention, the antisense sequence comprises AGUCUCUGGAUAUUCUCUC (SEQ ID NO:47), UUUAUUGGCAGCCUGAUCG (SEQ ID NO:48), UUGCUGAUCACUUCUGCGG (SEQ ID NO:49), CUGGAUAUUCUCUCUGGCA (SEQ ID NO:50), UCUGCCACUCCUGCCCCAU (SEQ ID NO:51), or GCCACUCCUGCCCCAUUUA (SEQ ID NO:69) and the antisense sequence hybridizes to a portion of mRNA corresponding to SEQ ID NO:2.

The above-cited method includes embodiments where the antisense sequence comprises AACCCCUUGGAGAGCCUCC(SEQ ID NO:52), UGCCCAUGUCCCCAACCCC (SEQ ID NO:53), AUAGAGAUAUCUGUUUGAA (SEQ ID NO:54), CGAGCAUAGAGAUAUCUGU (SEQ ID NO:55), CUUUGGGCAGCAUCAUAGU (SEQ ID NO:56), AGACACCCCCAGGUCCUCU (SEQ ID NO:57), CCUGGAACGGCUGAUGAGU (SEQ ID NO:58), CCAAAUAAAUAGUAGUCUA (SEQ ID NO:59), UCCAAUACAGUGCUGCUGU (SEQ ID NO:60), CUCAGCUUUCUCGUUGGAC (SEQ ID NO:61), CCAUUCCUCAGCUUUCUCG (SEQ ID NO:62), CCGGCCCCAUUCCUCAGCU (SEQ ID NO:63), CUUUGCCACUCCGGCCCCA (SEQ ID NO:64), or UCUGAAGCGGUCGGGGUCU (SEQ ID NO:65), and the antisense sequence hybridizes to a portion of mRNA corresponding to SEQ ID NO:3.

The antisense sequence of the siRNA has at least near-perfect contiguous complementarity of at least 19 nucleotides with the target sequence of the mRNA. "Near-perfect," as used herein, means the antisense sequence of the siRNA is "substantially complementary to," and the sense sequence of the siRNA is "substantially identical" to at least a portion of the target mRNA. "Identity," as known by one of ordinary skill in the art, is the degree of sequence relatedness between nucleotide sequences as determined by matching the order of nucleotides between the sequences. In one embodiment, antisense RNA having 80% and between 80% up to 100% complementarity to the target mRNA sequence are considered near-perfect complementarity and may be used in the present invention. "Perfect" contiguous complementarity is standard Watson-Crick base pairing of adjacent base pairs. "At least near-perfect" contiguous complementarity includes "perfect" complementarity as used herein. Computer methods for determining identity or complementarity are designed to provide the greatest degree of matching of nucleotide sequences, for example, BLASTP and BLASTN (Altschul, S.F., et al. (1990) J. Mol. Biol. 215:403-410), and FASTA.

The target sequence of mRNA corresponding to SEQ ID NO:1, SEQ ID NO:2, or SEQ ID NO:3 may be in the 5' or 3' untranslated regions of the mRNA as well as in the coding region of the mRNA.

One or both of the strands of double-stranded interfering RNA may have a 3' overhang of from 1 to 6 nucleotides which may be ribonucleotides or deoxyribonucleotides or a mixture thereof. The nucleotides of the overhang are not base-paired. In one embodiment of the invention, the interfering ds RNA comprises a 3' overhang of TT or UU.

The sense and antisense strands of the double stranded siRNA may be in a duplex formation of two single strands as described above or may be a single molecule where the regions of complementarity are base-paired and are covalently linked by a hairpin or loop so as to form a single strand. It is believed that the hairpin is cleaved intracellularly by a protein termed Dicer to form an interfering RNA of two individual base-paired RNA molecules.

Interfering RNAs may differ from naturally-occurring RNA by the addition, deletion, substitution or modification of one or more nucleotides. Non-nucleotide material may be bound to the interfering RNA, either at the 5' end, the 3' end, or internally. Such modifications are commonly designed to increase the nuclease resistance of the interfering RNAs, to improve cellular uptake, to enhance cellular targeting, to assist in tracing the interfering RNA, or to further improve stability. For example, interfering RNAs may comprise a purine nucleotide at the ends of overhangs. Conjugation of cholesterol to the 3' end of the sense strand of a ds siRNA molecule by means of a pyrrolidine linker, for example, also provides stability to an siRNA. Further modifications include a 3' terminal biotin molecule, a peptide known to have cell-penetrating properties, a nanoparticle, a peptidomimetic, a fluorescent dye, or a dendrimer, for example.

Nucleotides may be modified on their base portion, on their sugar portion, or on the phosphate portion of the molecule and function in embodiments of the present invention. Modifications include substitutions with alkyl, alkoxy, amino, deaza, halo, hydroxyl, thiol groups, or a combination thereof, for example. Nucleotides may be substituted with analogs with greater stability such as replacing U with 2'deoxy-T, or having a sugar modification such as a 2'OH replaced by a 2' amino or 2' methyl group, 2'methoxyethyl groups, or a 2'-0, 4'-C methylene bridge, for example. Examples of a purine or pyrimidine analog of nucleotides include a xanthine, a hypoxanthine, an azapurine, a methylthioadenine, 7-deaza-adenosine and O- and N-modified nucleotides. The phosphate group of the nucleotide may be modified by substituting one or more of the oxygens of the phosphate group with nitrogen or with sulfur (phosphorothioates). Modifications are useful for improving function, for example, for improving stability or permeability, or for localization or targeting.

There may be a region of the antisense siRNA that is not complementary to a portion of SEQ ID NO:1. Non-complementary regions may be at the 3', 5' or both ends of a complementary region.

Interfering RNAs may be synthetically generated, generated by *in vitro* transcription, siRNA expression vectors, or PCR expression cassettes, for example. Interfering RNAs that function well as transfected siRNAs also function well as siRNAs expressed *in vivo.*

Interfering RNAs are chemically synthesized using protected ribonucleoside phosphoramidites and a conventional DNA/RNA synthesizer and may be obtained from commercial suppliers such as Ambion Inc. (Austin, Texas), Invitrogen (Carlsbad, CA), or Dharmacon (Lafayette, Colo., USA), for example. Interfering RNAs are purified by extraction with a solvent or resin, precipitation, electrophoresis, chromatography, or a combination thereof, for example. Alternatively, interfering RNA may be used with little if any purification to avoid losses due to sample processing.

Interfering RNA may be provided to a subject by expression from a recombinant plasmid using a constitutive or inducible promoter such as the U6 or H1 RNA pol III promoter, the cytomegalovirus promoter, SP6, T3, or T7 promoter, known to those of ordinary skill in the art. For example, the psiRNA™ from InvivoGen (San Diego, CA) allows production of siRNAs within cells from an RNA pol III promoter. Interfering RNA expressed from recombinant plasmids may be isolated by standard techniques.

A viral vector for expression of interfering RNA may be derived from adenovirus, adeno-associated virus, vaccinia virus, retroviruses (lentiviruses, Rhabdoviruses, murine leukemia virus, for example), herpes virus, or the like, using promoters as cited above, for example, for plasmids. Selection of viral vectors, methods for expressing the interfering RNA by the vector and methods of delivering the viral vector are within the ordinary skill of one in the art.

Expression of interfering RNAs is also provided by use of SILENCER EXPRESS™ (Ambion, Austin, Texas) via expression cassettes (SECs) with a human H1, human U6 or mouse U6 promoter by PCR. Silencer expression cassettes are PCR products that include promoter and terminator sequences flanking a hairpin siRNA template. Upon transfection into cells, the hairpin siRNA is expressed from the PCR product and induces specific silencing.

*Hybridization under Physiological Conditions:* "Hybridization" refers to a technique where single-stranded nucleic acids (DNA or RNA) are allowed to interact so that hydrogen-bonded complexes called hybrids are formed by those nucleic acids with complementary or near-complementary base sequences. Hybridization reactions are sensitive and selective so that a particular sequence of interest is identified in samples in which it is present at low concentrations. The specificity of hybridization (i.e., stringency) is controlled by the concentrations of salt or formamide in the prehybridization and hybridization solutions *in vitro,* for example, and by the hybridization temperature, and are well known in the art. In particular, stringency is increased by reducing the concentration of salt, increasing the concentration of formamide, or raising the hybridization temperature.

For example, high stringency conditions could occur at about 50% formamide at 37 °C to 42 °C. Reduced stringency conditions could occur at about 35% to 25% formamide at about 30 °C to 35 °C. Examples of stringency conditions for hybridization are provided in Sambrook, J., 1989, Molecular Cloning: A Laboratory Manual, Cold Spring Harbor Laboratory Press, Cold Spring Harbor, N.Y. Further examples of stringent hybridization conditions include 400 mM NaCl, 40 mM PIPES pH 6.4, 1 mM EDTA, 50 °C or 70 °C for 12-16 hours followed by washing, or hybridization at 70 °C in 1XSSC or 50 °C in 1XSSC, 50% formamide followed by washing at 70 °C in 0.3XSSC, or hybridization at 70 °C in 4XSSC or 50 °C in 4XSSC, 50% formamide followed by washing at 67 °C in 1XSSC. The temperature for hybridization is about 5-10 °C less than the melting temperature (Tₘ) of the hybrid where Tₘ is determined for hybrids between 19 and 49 base pairs in length using the following calculation: Tₘ °C = 81.5 + 16.6(log₁₀[Na+]) + 0.41 (% G+C) - (600/N) where N is the number of bases in the hybrid, and [Na+] is the concentration of sodium ions in the hybridization buffer.

In embodiments of the present invention, an antisense strand of an interfering RNA that hybridizes with SAA mRNA *in vitro* under high stringency conditions will bind specifically *in vivo* under physiological conditions. Identification or isolation of a related nucleic acid that does not hybridize to a nucleic acid under highly stringent conditions is carried out under reduced stringency.

*Single stranded interfering RNA:* As cited above, interfering RNAs ultimately function as single strands. SS siRNA has been found to effect mRNA silencing, albeit less efficiently than double-stranded RNA. Therefore, embodiments of the present invention also provide for administration of ss siRNA where the single stranded siRNA hybridizes under physiological conditions to a portion of SEQ ID NO:1, SEQ ID NO:2, or SEQ ID NO:3, and has a region of at least near-perfect contiguous complementarity of at least 19 nucleotides with the hybridizing portion of SEQ ID NO:1, SEQ ID NO:2, or SEQ ID NO:3, respectively. The ss siRNA has a length of 19 to 49 nucleotides as for the ds siRNA cited above. The ss siRNA has a 5' phosphate or is phosphorylated *in situ* or *in vivo* at the 5' position. The term "5' phosphorylated" is used to describe, for example, polynucleotides or oligonucleotides having a phosphate group attached via ester linkage to the C5 hydroxyl of the 5' sugar (e.g., the 5' ribose or deoxyribose, or an analog of same). The ss siRNA may have a mono-, di-, or triphosphate group.

SS siRNAs are synthesized chemically or via vectors as for ds siRNAs. 5' Phosphate groups may be added via a kinase, or a 5' phosphate may be the result of nuclease cleavage of an RNA. Delivery is as for ds siRNAs. In one embodiment, ss siRNAs having protected ends and nuclease resistant modifications are administered for silencing. SS siRNAs may be dried for storage or dissolved in an aqueous solution. The solution may contain buffers or salts to inhibit annealing or for stabilization.

*Hairpin interfering RNA:* A hairpin interfering RNA is single-stranded and contains both the sense and antisense sequence within the one strand. For expression by a DNA vector, the corresponding DNA oligonucleotides of at least 19-nucleotides corresponding to the sense siRNA sequence are linked to its reverse complementary antisense sequence by a short spacer. If needed for the chosen expression vector, 3' terminal T's and nucleotides forming restriction sites may be added. The resulting RNA transcript folds back onto itself to form a stem-loop structure.

*Mode of administration:* Interfering RNA may be delivered directly to the eye by ocular tissue injection such as periocular, conjunctival, sub-Tenons, intracameral, intravitreal, sub-retinal, retrobulbar, or intracanalicular injections; by direct application to the eye using a catheter or other placement device such as a retinal pellet, intraocular insert, suppository or an implant comprising a porous, non-porous, or gelatinous material; by topical ocular drops or ointments; by a slow release device in the cul-de-sac or implanted adjacent to the sclera (transscleral) or within the eye. Intracameral injection may be through the cornea into the anterior chamber to allow the agent to reach the trabecular meshwork. Intracanalicular injection may be into the venous collector channels draining Schlemm's canal or into Schlemm's canal.

*Subject:* A subject in need of treatment for glaucoma or at risk for developing glaucoma is a human or other mammal having a condition or at risk of having glaucoma associated with expression or activity of SAA, i.e., an SAA-associated glaucoma. Ocular structures associated with such disorders may include the retina, choroid, lens, cornea, trabecular meshwork, iris, optic nerve, optic nerve head, sclera, aqueous chamber, vitreous chamber, or ciliary body, for example.

*Formulations and Dosage:* Pharmaceutical formulations comprise an interfering RNA, or salt thereof, of the invention up to 99% by weight mixed with a physiologically acceptable ophthalmic carrier medium such as water, buffer, saline, glycine, hyaluronic acid, mannitol, and the like.

Interfering RNAs of the present invention are administered as solutions, suspensions, or emulsions. The following are examples of possible formulations embodied by this invention.

| | Amount in weight % |
|---|---|
| Interfering RNA | up to 99; 0.1-99; 0.1 - 50; 0.5 -10.0 |
| Hydroxypropylmethylcellulose | 0.5 |
| Sodium chloride | .8 |
| Benzalkonium Chloride | 0.01 |
| EDTA | 0.01 |
| NaOH/HCl | qs pH 7.4 |
| Purified water | qs 100 mL |

| | Amount in weight % |
|---|---|
| Interfering RNA | up to 99; 0.1-99; 0.1 - 50; 0.5 - 10.0 |
| Phosphate Buffered Saline | 1.0 |
| Benzalkonium Chloride | 0.01 |
| Polysorbate 80 | 0.5 |
| Purified water | q.s. to 100% |

| | |
|---|---|
| Interfering RNA | up to 99; 0. 1-99; 0.1 - 50; 0.5 - 10.0 |
| Monobasic sodium phosphate | 0.05 |
| | Amount in weight % |
| Dibasic sodium phosphate (anhydrous) | 0.15 |
| Sodium chloride | 0.75 |

| | |
|---|---|
| Disodium EDTA | 0.05 |
| Cremophor EL | 0.1 |
| Benzalkonium chloride | 0.01 |
| HCl and/or NaOH | pH 7.3-7.4 |
| Purified water | q.s. to 100% |

| | Amount in weight % |
|---|---|
| Interfering RNA | up to 99; 0.1-99; 0.1 - 50; 0.5 - 10.0 |
| Phosphate Buffered Saline | 1.0 |
| Hydroxypropyl-β-cyclodextrin | 4.0 |
| Purified water | q.s. to 100% |

Generally, an effective amount of the interfering RNA of embodiments of the invention comprises an intercellular concentration at or near the ocular site of from 200pM to 100 nM, or from 1 nM to 50 nM, or from 5 nM to about 25 nM. Topical compositions are delivered to the surface of the eye one to four times per day according to the routine discretion of a skilled clinician. The pH of the formulation is about pH 4-9, or pH 4.5 to pH 7.4.

While the precise regimen is left to the discretion of the clinician, interfering RNA may be administered by placing one drop in each eye one to four times a day, or as directed by the clinician. An effective amount of a formulation may depend on factors such as the age, race, and sex of the subject, or the severity of the glaucoma, for example. In one embodiment, the interfering RNA is delivered topically to the eye and reaches the trabecular meshwork, retina or optic nerve head at a therapeutic dose thereby ameliorating an SAA-associated disease process.

*Acceptable carriers:* An ophthalmically acceptable carrier refers to those carriers that cause at most, little to no ocular irritation, provide suitable preservation if needed, and deliver one or more interfering RNAs of the present invention in a homogenous dosage. An acceptable carrier for administration of interfering RNA of embodiments of the present invention include the Mirus TransIT^{®}-TKO siRNA Tranfection Reagent (Mirus Corporation, Madison, Wisconsin), LIPOFECTIN®, lipofectamine, OLIGOFECTAMINE™ (Invitrogen, Carlsbad, CA), CELLFECTIN®, DHARMAFECT™ (Dharmacon, Chicago, IL) or polycations such as polylysine, liposomes, or fat-soluble agents such as cholesterol. Liposomes are formed from standard vesicle-forming lipids and a sterol, such as cholesterol, and may include a targeting molecule such as a monoclonal antibody having binding affinity for endothelial cell surface antigens, for example. Further, the liposomes may be PEGylated liposomes.

For ophthalmic delivery, an interfering RNA may be combined with ophthalmologically acceptable preservatives, co-solvents, surfactants, viscosity enhancers, penetration enhancers, buffers, sodium chloride, or water to form an aqueous, sterile ophthalmic suspension or solution. Ophthalmic solution formulations may be prepared by dissolving the inhibitor in a physiologically acceptable isotonic aqueous buffer. Further, the ophthalmic solution may include an ophthalmologically acceptable surfactant to assist in dissolving the inhibitor. Viscosity building agents, such as hydroxymethyl cellulose, hydroxyethyl cellulose, methylcellulose, polyvinylpyrrolidone, or the like, may be added to the compositions of the present invention to improve the retention of the compound.

In order to prepare a sterile ophthalmic ointment formulation, the interfering RNA is combined with a preservative in an appropriate vehicle, such as mineral oil, liquid lanolin, or white petrolatum. Sterile ophthalmic gel formulations may be prepared by suspending the interfering RNA in a hydrophilic base prepared from the combination of, for example, CARBOPOL^{®}-940 (BF Goodrich, Charlotte, NC), or the like, according to methods known in the art for other ophthalmic formulations. VISCOAT^{®} (Alcon Laboratories, Inc., Fort Worth, TX) may be used for intraocular injection, for example. Other compositions of the present invention may contain penetration enhancing agents such as cremephor and TWEEN^{®} 80 (polyoxyethylene sorbitan monolaureate, Sigma Aldrich, St. Louis, MO), in the event the interfering RNA is less penetrating in the eye.

*Kits:* Embodiments of the present invention provide a kit that includes reagents for attenuating the expression of an SAA mRNA in a cell. The kit contains a DNA template that has two different promoters such as a T7 promoter, a T3 promoter or an SP6 promoter, each operably linked to a nucleotide sequence that encodes two complementary single-stranded RNAs corresponding to an interfering RNA. RNA is transcribed from the DNA template and is annealed to form a double-stranded RNA effective to attenuate expression of the target mRNA. The kit optionally contains amplification primers for amplifying the DNA sequence from the DNA template and nucleotide triphosphates (i.e., ATP, GTP, CTP and UTP) for synthesizing RNA. Optionally, the kit contains two RNA polymerases, each capable of binding to a promoter on the DNA template and effecting transcription of the nucleotide sequence to which the promoter is operably linked, a purification column for purifying single-stranded RNA, such as a size exclusion column, one or more buffers, for example, a buffer for annealing single-stranded RNAs to yield double stranded RNA, and RNAse A or RNAse T for purifying double stranded RNA.

The ability of SAA interfering RNA to knock-down the levels of endogenous SAA expression in, for example, human trabecular meshwork (TM) cells is carried out as follows. Transfection of a transformed human TM cell line designated GTM3 or HTM-3 (see Pang, I.H. et al.,. 1994. Curr. Eye Res. 13:51-63) is accomplished using standard *in vitro* concentrations of SAA interfering RNA (100 nM) as cited herein and LIPOFECTAMINE™ 2000 (Invitrogen, Carlsbad, California) at a 1:1 (w/v) ratio. Scrambled and lamin A/C siRNA (Dharmacon) are used as controls.

QPCR TAQMAN® forward and reverse primers and a probe set that encompasses the target site are used to assess the degree of mRNA cleavage. Such primer/probe sets may be synthesized by ABI (Applied Biosystems, Foster City, CA), for example.

To reduce the chance of non-specific, off-target effects, the lowest possible siRNA concentration for inhibiting SAA mRNA expression is determined for an siRNA. SAA mRNA knock-down is assessed by QPCR amplification using an appropriate primer/probe set. A dose response of SAA siRNA in GTM3 cells is observed in GTM3 cells after 24 hour treatment with 0, 1, 3, 10, 30, and 100 nM dose range of siRNA, for example. Data are fitted using GraphPad Prism 4 software (GraphPad Software, Inc., San Diego, CA) with a variable slope, sigmoidal dose response algorithm and a top constraint of 100%. An IC₅₀ is obtained for the particular siRNA tested.

### Example 1

### Interfering RNA for Silencing SAA in Trabecular Meshwork Cells

The present study examines the ability of SAA-interfering RNA to knock-down the levels of endogenous SAA expression in normal and glaucomatous human trabecular meshwork (TM) cells.

Transfection of a normal (NTM765-04-OD, p5) and a glaucomatous (GTM686-03-OS, p6) TM cell line was carried out using standard *in vitro* concentrations of a SMARTPOOL® SAA-interfering RNA pool (100 nM) and DHARMAFECT® #1 transfection reagent (Dharmacon Research Inc., Chicago, IL). The SMARTPOOL® SAA-interfering RNA contained a pool of four homologous, double-stranded siRNAs designed to target SAA mRNA regions having the sequence identifiers SEQ ID NO:11, SEQ ID NO:18, SEQ ID NO:19, and SEQ ID NO:20 and was used at three different concentrations (Treatment 1: 0.05µl/100µl well; Treatment 2: 0.2µl/100µl well; Treatment 3: 0.4 µl/100µl well) in triplicate for 24 or 48 hr. The control had no treatment.

*Effects on mRNA Levels:* For QPCR analysis of SAA mRNA, total RNA was extracted from the 24 hr treated cells using RNAqueous-4™ PCR (Ambion, Austin, TX) and cDNA was synthesized with TaqMan® reverse transcription agents (PE Biosystems, Foster City, CA). The QPCR was performed using TaqMan® universal PCR master mix and 7700 SDS (PE Biosystems) in triplicate. Ribosomal RNA (18s rRNA, PE Biosystems) was used as a normalization control in the multiplex QPCR. QPCR analyses were conducted using two sets of TaqMan® probe/primers (PE Biosystems). A first set (P423) targets the coding region of SAA cDNA sequence and a second set (P428) targets the non-coding region.

As shown in FIG. 1, an about 35% inhibition of SAA mRNA relative to 18s rRNA was observed in siRNA treated NTM765-04 normal cells under conditions of Treatment 3 using the P423 primer set. As shown in FIG. 2, an about 41% inhibition of SAA mRNA relative to 18s rRNA was observed in siRNA treated GTM686-03 glaucomatous cells under conditions of Treatment 3 using the P423 primer set. Similar results were obtained using the primer set P428.

*Effects on SAA Protein Levels:* ELISA assays were used to examine the levels of endogenous SAA protein in cell lysates prepared from the 48 hr treated cells.

An about 66% decrease of SAA protein was observed in all of Treatment 1, Treatment 2, and Treatment 3 siRNA treated GTM 686 glaucomatous cells (FIG. 5) but not in NTM765 cells (FIG. 4). The endogenous SAA protein level was very low in both trabecular meshwork cell lines, particularly in the NTM765 normal cell line.

*Effects on Cell Growth and Morphology:* The effect of the SAA siRNA on TM cell morphology was monitored by a real time electronic sensing system (RT-CES™, ACEA Biosciences, Inc., San Diego, CA). As shown in FIG. 3, no toxic effects were observed due to the siRNA treatments on the growth or the morphology of TM cells.

Those of skill in the art, in light of the present disclosure, will appreciate that obvious modifications of the embodiments disclosed herein can be made without departing from the spirit and scope of the invention. All of the embodiments disclosed herein can be made and executed without undue experimentation in light of the present disclosure. The full scope of the invention is set out in the disclosure and equivalent embodiments thereof. The specification should not be construed to unduly narrow the full scope of protection to which the present invention is entitled.

As used herein and unless otherwise indicated, the terms "a" and "an" are taken to mean "one", "at least one" or "one or more".

## Claims

1. A composition comprising an effective amount of interfering RNA having a length of 19 to 49 nucleotides and a pharmaceutically acceptable carrier, the interfering RNA comprising: a sense nucleotide sequence, an antisense nucleotide sequence, and a region of at least 80% contiguous complementarity of at least 19 nucleotides between the sense and antisense sequences; wherein the antisense sequence hybridizes under physiological conditions to a portion of mRNA corresponding to SEQ ID NO: 1 or SEQ ID NO: 2 , and has a region of at least 80% contiguous complementarity of at least 19 nucleotides with the hybridizing portion of mRNA corresponding to SEQ ID NO: 1 or SEQ ID NO: 2 for use in treating serum amyloid A-associated glaucoma in an eye of a subject.

2. A composition comprising an effective amount of interfering RNA having a length of 19 to 49 nucleotides and a pharmaceutically acceptable carrier, the interfering RNA comprising: a nucleotide sequence having a region of at least 80% contiguous complementarity of at least 19 nucleotides with a hybridizing portion of mRNA corresponding to SEQ ID NO: 1 or SEQ ID NO: 2, wherein the nucleotide sequence hybridizes under physiological conditions to a portion of mRNA corresponding to SEQ ID NO: 1 or SEQ ID NO: 2, for use in treating amyloid A-associated glaucoma in an eye of a subject.

3. An *in vitro* method for attenuating expression of serum amyloid A mRNA comprising administering an effective amount of interfering RNA having a length of 19 to 49 nucleotides and a pharmaceutically acceptable carrier, the interfering RNA comprising: a sense nucleotide sequence, an antisense nucleotide sequence, and a region of at least 80% contiguous complementarity of at least 19 nucleotides between the sense and antisense sequences; wherein the antisense sequence hybridizes under physiological conditions to a portion of mRNA corresponding to SEQ ID NO: 1 or SEQ ID NO:2, and has a region of at least 80% contiguous complementarity of at least 19 nucleotides with the hybridizing portion of mRNA corresponding to SEQ ID NO: 1 or SEQ ID NO:2 to a cell.

4. An *in vitro* method for attenuating expression of serum amyloid A mRNA comprising administering an effective amount of interfering RNA having a length of 19 to 49 nucleotides and a pharmaceutically acceptable carrier, the interfering RNA comprising: a nucleotide sequence having a region of at least 80% contiguous complementarity of at least 19 nucleotides with a hybridizing portion of mRNA corresponding to SEQ ID NO: 1 or SEQ ID NO:2, wherein the nucleotide sequence hybridizes under physiological conditions to a portion of mRNA corresponding to SEQ ID NO: 1 or SEQ ID NO: 2 to a cell.

5. The composition or method of Claim 1 or 3, wherein the antisense sequence has a region of at least 80% contiguous complementarity of at least 21 to 23 nucleotides with the hybridizing portion of mRNA corresponding to SEQ ID NO: 1 or SEQ ID NO: 2, and comprises an additional TT sequence at the 3' end of each of the sense and the antisense sequence.

6. The composition or method of Claim 1, 3 or 5, wherein the sense nucleotide sequence and the antisense nucleotide sequence are connected by a loop nucleotide sequence.

7. The composition or method of any of claims 1, 3, 5 and 6 wherein the antisense sequence is designed to target a nucleotide sequence of mRNA corresponding to SEQ ID NO: 1 beginning at nucleotide 230, 357, 362, 380, 447, 470, 527, 531, 548, or 557.

8. The composition or method of any of claims 1, 3, 5 and 6 wherein the antisense sequence is designed to target a nucleotide sequence of mRNA corresponding to SEQ ID NO: 2 beginning at nucleotide 43, 170, 175, 193, 260, 283, 339, or 370.

9. The composition or method of any of claims 1, 3, 5, 6 and 8 wherein the antisense sequence is designed to target a nucleotide sequence of mRNA corresponding to SEQ ID NO:2 beginning at nucleotide 252, 271, 276, 325, 343.

10. The composition or method of any of claims 1, 3, 5 to 9 wherein the antisense sequence comprises
CUUUGCCACUCCUGCCCCA (SEQ ID NO:37), UCGGAAGUGAUUGGGGUCU (SEQ ID NO:38),
UUUGUCUGAGCCGAUGUAA (SEQ ID NO:39), AACCAGGCCCGUGAGAAGC (SEQ ID NO:40),
CUGAGCCGAUGUAAUUGGC (SEQ ID NO:41), GCCACUCCUGCCCCAUUUA (SEQ ID NO:69),
CCCCCGAGCAUGGAAGUAU (SEQ ID NO:42), CUCUGGCAUUGCUGAUCAC (SEQ ID NO:43),
GCCUGUGAGUCUCUGGAUA (SEQ ID NO:44), GCCACUCCUGCCCCAUUUA (SEQ ID NO:45),
GCCAGCAGGUCGGAAGUGA (SEQ ID NO: 46), AGUCUCUGGAUAUUCUCUC(SEQ ID NO:47),
UUUAUUGGCAGCCUGAUCG(SEQIDNO:48), UUGCUGAUCACUUCUGCGG(SEQIDNO:49),
CUGGAUAUUCUCUCUGGCA(SEQIDNO:50), UCUGCCACUCCUGCCCCAU(SEQIDNO: 51),
AACCCCUUGGAGAGCCUCC(SEQIDNO:52), UGCCCAUGUCCCCAACCCC(SEQIDNO:53),
AUAGAGAUAUCUGUUUGAA(SEQIDNO:54), CGAGCAUAGAGAUAUCUGU(SEQIDNO:55),
CUUUGGGCAGCAUCAUAGU(SEQIDNO:56), AGACACCCCCAGGUCCUCU(SEQIDNO:57),
CCUGGAACGGCUGAUGAGU(SEQIDNO:58), CCAAAUAAAUAGUAGUCUA(SEQIDNO:59),
UCCAAUACAGUGCUGCUGU(SEQIDNO:60), CUCAGCUUUCUCGUUGGAC(SEQIDNO:61),
CCAUUCCUCAGCUUUCUCG(SEQIDNO:62), CCGGCCCCAUUCCUCAGCU(SEQIDNO:63),
CUUUGCCACUCCGGCCCCA(SEQIDNO:64),or UCUGAAGCGGUCGGGGUCU(SEQIDNO:65).

11. The composition or method of any preceding claim wherein the interfering RNA comprises a modification on a base portion, on a sugar portion or on a phosphate portion.

12. The composition or method of any of claims 1, 3 and 5, wherein the composition further comprises a second interfering RNA having a length of 19 to 49 nucleotides, and comprising a sense nucleotide sequence, an antisense nucleotide sequence, and a region of at least 80% complementarity of at least 19 nucleotides between the sense and antisense sequences; wherein the antisense sequence of the second interfering RNA hybridizes under physiological conditions to a second portion of mRNA corresponding to SEQ ID NO: 1 or SEQ ID NO: 2 and the antisense sequence has a region of at least 80% contiguous complementarity of at least 19 nucleotides with the second hybridizing portion of mRNA corresponding to SEQ ID NO: 1 or SEQ ID NO: 2.

13. The composition of claim 1, 3 and 5, wherein the composition comprises an effective amount of a mixture of at least four interfering RNAs, each interfering RNA having a length of 19 to 49 nucleotides, and a pharmaceutically acceptable carrier, each interfering RNA comprising: a sense nucleotide sequence, an antisense nucleotide sequence, and a region of at least 80% contiguous complementarity of at least 19 nucleotides between the sense and antisense sequences of each of the four interfering RNAs; wherein the antisense sequences of the mixture hybridize under physiological conditions to a portion of mRNA corresponding to SEQ ID NO: 2 beginning at nucleotide 175, 252, 276, and 325, respectively, and have a region of at least 80% contiguous complementarity of at least 19 nucleotides with the hybridizing portion of mRNA corresponding to SEQ ID NO: 2 beginning at nucleotide 175, 252, 276, and 325, respectively.

14. The composition of Claim 2 or the method of claim 4 wherein the composition further comprises a second interfering RNA having a length of 19 to 49 nucleotides, and comprising a second nucleotide sequence having a region of at least 80% contiguous complementarity of at least 19 nucleotides with a second hybridizing portion of mRNA corresponding to SEQ ID NO: 1 or SEQ ID NO: 2.

15. The composition of Claim 2 or the method of claim 4 wherein the composition comprises an effective amount of a mixture of at least four interfering RNAs, each interfering RNA having a length of 19 to 49 nucleotides, and the mixture comprising: a first, second, third and fourth nucleotide sequence having a region of at least 80 % contiguous complementarity of at least 19 nucleotides with the hybridizing portion of mRNA corresponding to SEQ ID NO: 2 beginning at nucleotide 175, 252, 276, and 325, respectively.

16. The composition or method of any preceding claim wherein the composition is prepared for administration via a topical, intravitreal, or transcleral route.

## Patentansprüche

1. Zusammensetzung umfassend eine wirksame Menge interferierender RNA mit einer Länge von 19 bis 49 Nukleotiden und einen pharmazeutisch verträglichen Träger, wobei die interferierender RNA umfasst: eine Sinn-Nukleotidsequenz, eine Antisinn-Nukleotidsequenz und eine Region von mindestens 80% kontinuierlicher Komplementarität von mindestens 19 Nukleotiden zwischen den Sinn- und Antisinn-Sequenzen; wobei die Antisinn-Sequenz unter physiologischen Bedingungen an einen Teil der mRNA hybridisiert, die SEQ ID Nr: 1 oder SEQ ID Nr: 2 entspricht, und eine Region von mindestens 80% kontinuierlicher Komplementarität von mindestens 19 Nukleotiden mit dem hybridisierenden Teil der mRNA aufweist, die SEQ ID Nr: 1 oder SEQ ID Nr: 2 entspricht, zur Verwendung bei der Behandlung von Serum-Amyloid A-assoziierten Glaukom im Auge eines Patienten.

2. Zusammensetzung umfassend eine wirksame Menge interferierender RNA mit einer Länge von 19 bis 49 Nukleotiden und einen pharmazeutisch verträglichen Träger, wobei die interferierender RNA umfasst: eine Nukleotidsequenz mit einer Region von mindestens 80% kontinuierlicher Komplementarität von mindestens 19 Nukleotiden mit dem hybridisierenden Teil der mRNA aufweist, die SEQ ID Nr: 1 oder SEQ ID Nr: 2 entspricht, wobei die Nukleotidsequenz unter physiologischen Bedingungen an einen Teil der mRNA hybridisiert, die SEQ ID Nr: 1 oder SEQ ID Nr: 2 entspricht, zur Verwendung bei der Behandlung von Serum-Amyloid A-assoziierten Glaukom im Auge eines Patienten.

3. *In vitro* Verfahren zum Abschwächen der Expression von Serum-Amyloid A mRNA umfassend das Verabreichen einer wirksamen Menge der interferierenden RNA mit einer Länge von 19 bis 49 Nukleotiden und einen pharmazeutisch verträglichen Träger an eine Zelle, wobei die interferierender RNA umfasst: eine Sinn-Nukleotidsequenz, eine Antisinn-Nukleotidsequenz und eine Region von mindestens 80% kontinuierlicher Komplementarität von mindestens 19 Nukleotiden zwischen den Sinn- und Antisinn-Sequenzen; wobei die Antisinn-Sequenz unter physiologischen Bedingungen an einen Teil der mRNA hybridisiert, die SEQ ID Nr: 1 oder SEQ ID Nr: 2 entspricht, und eine Region von mindestens 80% kontinuierlicher Komplementarität von mindestens 19 Nukleotiden mit dem hybridisierenden Teil der mRNA aufweist, die SEQ ID Nr: 1 oder SEQ ID Nr: 2 entspricht.

4. *In vitro* Verfahren zum Abschwächen der Expression von Serum-Amyloid A mRNA umfassend das Verabreichen einer wirksamen Menge der interferierenden RNA mit einer Länge von 19 bis 49 Nukleotiden und einen pharmazeutisch verträglichen Träger an eine Zelle, wobei die interferierender RNA umfasst: eine Nukleotidsequenz mit einer Region von mindestens 80% kontinuierlicher Komplementarität von mindestens 19 Nukleotiden mit dem hybridisierenden Teil der mRNA, die SEQ ID Nr: 1 oder SEQ ID Nr: 2 entspricht, wobei die Nukleotidsequenz unter physiologischen Bedingungen an einen Teil der mRNA hybridisiert, die SEQ ID Nr: 1 oder SEQ ID Nr: 2 entspricht.

5. Zusammensetzung oder Verfahren nach Anspruch 1 oder 3, wobei die Antisinn-Sequenz eine Region von mindestens 80% kontinuierlicher Komplementarität von mindestens 21 bis 23 Nukleotiden mit dem hybridisierenden Teil der mRNA aufweist, die SEQ ID Nr: 1 oder SEQ ID Nr: 2 entspricht, und eine zusätzliche TT -Sequenz am 3'-Ende jeweils der Sinn- und der Antisinn-Sequenz aufweist.

6. Zusammensetzung oder Verfahren nach Anspruch 1, 3 oder 5, worin die Sinn-Nukleotidsequenz und die Antisinn-Nukleotidsequenz durch eine Schleifen-Nukleotidsequenz verbunden sind.

7. Zusammensetzung oder Verfahren nach einem der Ansprüche 1, 3, 5 und 6, wobei die Antisinn-Sequenz so gestaltet ist, dass sie auf eine Nukleotidsequenz einer mRNA abzielt, die der SEQ ID Nr: 1 beginnend bei Nukleotid 230, 357, 362, 380, 447, 470, 527, 531, 548 oder 557 entspricht.

8. Zusammensetzung oder Verfahren nach einem der Ansprüche 1, 3, 5 und 6, wobei die Antisinn-Sequenz so gestaltet ist, dass sie auf eine Nukleotidsequenz einer mRNA abzielt, die der SEQ ID Nr: 2 beginnend bei Nukleotid 43, 170, 175, 193, 260, 283, 339 oder 370 entspricht.

9. Zusammensetzung oder Verfahren nach einem der Ansprüche 1, 3, 5, 6 und 8, wobei die Antisinn-Sequenz so gestaltet ist, dass sie auf eine Nukleotidsequenz einer mRNA abzielt, die der SEQ ID Nr: 2 beginnend bei Nukleotid 252, 271, 276, 325 oder 343 entspricht.

10. Zusammensetzung oder Verfahren nach einem der Ansprüche 1, 3, 5 bis 9, wobei die Antisinn-Sequenz umfasst:
CUUUGCCACUCCUGCCCCA (SEQ ID Nr: 37), UCGGAAGUGAUUGGGGUCU (SEQ ID Nr: 38),
UUUGUCUGAGCCGAUGUAA (SEQ ID Nr: 39), AACCAGGCCCGUGAGAAGC (SEQ ID Nr: 40),
CUGAGCCGAUGUAAUUGGC (SEQ ID Nr: 41), GCCACUCCUGCCCCAUUUA (SEQ ID Nr: 69),
CCCCCGAGCAUGGAAGUAU (SEQ ID Nr: 42), CUCUGGCAUUGCUGAUCAC (SEQ ID Nr: 43),
GCCUGUGAGUCUCUGGAUA (SEQ ID Nr: 44), GCCACUCCUGCCCCAUUUA (SEQ ID Nr: 45),
GCCAGCAGGUCGGAAGUGA (SEQ ID Nr: 46), AGUCUCUGGAUAUUCUCUC (SEQ ID Nr: 47),
UUUAUUGGCAGCCUGAUCG (SEQ ID Nr: 48), UUGCUGAUCACUUCUGCGG (SEQ ID Nr: 49),
CUGGAUAUUCUCUCUGGCA (SEQ ID Nr: 50), UCUGCCACUCCUGCCCCAU (SEQ ID Nr: 51),
AACCCCUUGGAGAGCCUCC (SEQ ID Nr: 52), UGCCCAUGUCCCCAACCCC (SEQ ID Nr: 53),
AUAGAGAUAUCUGUUUGAA (SEQ ID Nr: 54), CGAGCAUAGAGAUAUCUGU (SEQ ID Nr: 55),
CUUUGGGCAGCAUCAUAGU (SEQ ID Nr: 56), AGACACCCCCAGGUCCUCU (SEQ ID Nr: 57),
CCUGGAACGGCUGAUGAGU (SEQ ID Nr: 58), CCAAAUAAAUAGUAGUCUA (SEQ ID Nr: 59),
UCCAAUACAGUGCUGCUGU( SEQ ID Nr: 60), CUCAGCUUUCUCGUUGGAC (SEQ ID Nr: 61),
CCAUUCCUCAGCUUUCUCG (SEQ ID Nr: 62), CCGGCCCCAUUCCUCAGCU (SEQ ID Nr: 63),
CUUUGCCACUCCGGCCCCA (SEQ ID Nr: 64) oder UCUGAAGCGGUCGGGGUCU (SEQ ID Nr: 65).

11. Zusammensetzung oder Verfahren nach einem der vorhergehenden Ansprüche, wobei die interferierende RNA eine Modifikation auf dem Basenanteil, Zuckeranteil oder Phosphatanteil umfasst.

12. Zusammensetzung oder Verfahren nach einem der Ansprüche 1, 3 und 5, wobei die Zusammensetzung weiterhin eine zweite interferierende RNA umfasst, die eine Länge von 19 bis 49 Nukleotide hat, und eine Sinn-Nukleotidsequenz, eine Antisinn-Nukleotidsequenz und eine Region von mindestens 80% kontinuierlicher Komplementarität von mindestens 19 Nukleotiden zwischen den Sinn- und Antisinn-Sequenzen umfasst; wobei die Antisinn-Sequenz der zweiten interferierenden RNA unter physiologischen Bedingungen an einen zweiten Anteil der mRNA hybridisiert, die SEQ ID Nr: 1 oder SEQ ID Nr: 2 entspricht und die Antisinn-Sequenz eine Region aufweist mit mindestens 80% kontinuierlicher Komplementarität von mindestens 19 Nukleotiden mit dem zweiten hybridisierenden Teil der mRNA, die SEQ ID Nr: 1 oder SEQ ID Nr: 2 entspricht.

13. Zusammensetzung nach einem der Ansprüche 1, 3 und 5, wobei die Zusammensetzung eine wirksame Menge einer Mischung von mindestens vier interferierenden RNAs umfasst, wobei jede interferierende RNA eine Länge von 19 bis 49 Nukleotiden aufweist, und einen pharmazeutisch verträglichen Träger, wobei jede interferierende RNA umfasst: eine Sinn-Nukleotidsequenz, eine Antisinn-Nukleotidsequenz und eine Region von mindestens 80% kontinuierlicher Komplementarität von mindestens 19 Nukleotiden zwischen den Sinn- und Antisinn-Sequenzen jeder der vier interferierenden RNAs umfasst; wobei die Antisinn-Sequenzen der Mischung unter physiologischen Bedingungen an einen Teil der mRNA hybridisieren, der SEQ ID Nr: 2, beginnend bei Nukleotid 175, 252, 276 beziehungsweise 325 entspricht, und eine Region haben mit mindestens 80% kontinuierlicher Komplementarität von mindestens 19 Nukleotiden mit dem hybridisierenden Teil der mRNA, die SEQ ID Nr: 2, beginnend bei Nukleotid 175, 252, 276, beziehungsweise 325 entspricht.

14. Zusammensetzung nach Anspruch 2 oder Verfahren nach Anspruch 4, wobei die Zusammensetzung weiterhin eine zweite interferierende RNA mit einer Länge von 19 bis 49 Nukleotiden und eine zweite Nukleotidsequenz umfasst, mit einer Region von mindestens 80% kontinuierlicher Komplementarität von mindestens 19 Nukleotiden mit einem zweiten hybridisierenden Teil der mRNA, die SEQ ID Nr: 1 oder SEQ ID Nr: 2 entspricht.

15. Zusammensetzung nach Anspruch 2 oder Verfahren nach Anspruch 4, wobei die Zusammensetzung eine wirksame Menge einer Mischung von mindestens vier interferierenden RNAs umfasst, wobei jede interferierende RNA eine Länge von 19 bis 49 Nukleotiden aufweist, und die Mischung umfasst: eine erste, zweite, dritte und vierte Nukleotidsequenz mit einer Region von mindestens 80% kontinuierlicher Komplementarität von mindestens 19 Nukleotiden mit dem hybridisierenden Teil der mRNA, die SEQ ID Nr: 2, beginnend bei Nukleotid 175, 252, 276 beziehungsweise 325 entspricht.

16. Zusammensetzung oder Verfahren nach einem der vorhergehenden Ansprüche, wobei die Zusammensetzung für die Verabreichung über den topischen, intravitrealen oder transkleralen Weg hergestellt ist.

## Revendications

1. Composition comprenant une quantité efficace d'ARN interférant d'une longueur de 19 à 49 nucléotides et un vecteur acceptable au plan pharmaceutique, l'ARN interférant comprenant : une séquence nucléotidique sens, une séquence nucléotidique anti-sens et une région d'au moins 80% de complémentarité contiguë d'au moins 19 nucléotides entre les séquences sens et anti-sens ; dans laquelle la séquence anti-sens s'hybride, dans les conditions physiologiques, à une partie de l'ARNm correspondant à la SEQ ID N°1 ou à la SEQ ID N°2, et a une région d'au moins 80% de complémentarité contiguë d'au moins 19 nucléotides avec la partie hybridante de l'ARNm correspondant à la SEQ ID N°1 ou à la SEQ ID N°2, pour usage dans le traitement du glaucome associé à la substance amyloïde A sérique dans l'oeil d'un sujet.

2. Composition comprenant une quantité efficace d'ARN interférant d'une longueur de 19 à 49 nucléotides et un vecteur acceptable au plan pharmaceutique, l'ARN interférant comprenant : une séquence nucléotidique ayant une région d'au moins 80% de complémentarité contiguë d'au moins 19 nucléotides avec une partie hybridante de l'ARNm correspondant à la SEQ ID N°1 ou à la SEQ ID N°2, dans laquelle la séquence nucléotidique s'hybride, dans les conditions physiologiques, à une partie de l'ARNm correspondant à la SEQ ID N°1 ou à la SEQ ID N°2, pour usage dans le traitement du glaucome associé à la substance amyloïde A dans l'oeil d'un sujet.

3. Méthode *in vitro* pour atténuer l'expression de l'ARNm de la substance amyloïde A sérique, comprenant l'administration à une cellule d'une quantité efficace d'ARN interférant d'une longueur de 19 à 49 nucléotides et un vecteur acceptable au plan pharmaceutique, l'ARN interférant comprenant : une séquence nucléotidique sens, une séquence nucléotidique anti-sens et une région d'au moins 80% de complémentarité contiguë d'au moins 19 nucléotides entre les séquences sens et anti-sens ; dans laquelle la séquence anti-sens s'hybride, dans les conditions physiologiques, à une partie de l'ARNm correspondant à la SEQ ID N°1 ou à la SEQ ID N°2 et a une région d'au moins 80% de complémentarité contiguë d'au moins 19 nucléotides avec la partie hybridante de l'ARNm correspondant à la SEQ ID N°1 ou à la SEQ ID N°2.

4. Méthode *in vitro* pour atténuer l'expression de l'ARNm de la substance amyloïde A sérique, comprenant l'administration à une cellule d'une quantité efficace d'ARN interférant d'une longueur de 19 à 49 nucléotides et un vecteur acceptable au plan pharmaceutique, l'ARN interférant comprenant : une séquence nucléotidique ayant une région d'au moins 80% de complémentarité contiguë d'au moins 19 nucléotides avec une partie hybridante de l'ARNm correspondant à la SEQ ID N°1 ou à la SEQ ID N°2, dans laquelle la séquence nucléotidique s'hybride, dans les conditions physiologiques, à une partie de l'ARNm correspondant à la SEQ ID N°1 ou à la SEQ ID N°2.

5. Composition ou méthode selon la revendication 1 ou 3, dans laquelle la séquence anti-sens a une région d'au moins 80% de complémentarité contiguë d'au moins 21 à 23 nucléotides avec la partie hybridante de l'ARNm correspondant à la SEQ ID N°1 ou à la SEQ ID N°2 et comprend une séquence TT additionnelle à l'extrémité 3' de chacune des séquences sens et anti-sens.

6. Composition ou méthode selon la revendication 1, 3 ou 5 dans laquelle la séquence nucléotidique sens et la séquence nucléotidique anti-sens sont connectées par une séquence nucléotidique en boucle.

7. Composition ou méthode selon l'une quelconque des revendications 1, 3, 5 et 6, dans laquelle la séquence anti-sens est conçue pour cibler une séquence nucléotidique d'ARNm correspondant à la SEQ ID N°1, commençant au nucléotide 230, 357, 362, 380, 447, 470, 527, 531, 548 ou 557.

8. Composition ou méthode selon l'une quelconque des revendications 1, 3, 5 et 6, dans laquelle la séquence anti-sens est conçue pour cibler une séquence nucléotidique d'ARNm correspondant à la SEQ ID N°2, commençant au nucléotide 43, 170, 175, 193, 260, 283, 339 ou 370.

9. Composition ou méthode selon l'une quelconque des revendications 1, 3, 5, 6 et 8, dans laquelle la séquence anti-sens est conçue pour cibler une séquence nucléotidique d'ARNm correspondant à la SEQ ID N°2, commençant au nucléotide 252, 271, 276, 325, 343.

10. Composition ou méthode selon l'une quelconque des revendications 1, 3, 5 à 9, dans laquelle la séquence anti-sens comprend
| | | |
|---|---|---|
| CUUUGCCACUCCUGCCCCA | (SEQ ID N°37), | UCGGAAGUGAUUGGGGUCU |
| (SEQ ID N°38), | UUUGUCUGAGCCGAUGUAA | (SEQ ID N°39), |
| AACCAGGCCCGUGAGAAGC | (SEQ ID N° 40), | CUGAGCCGAUGUAAUUGGC |
| (SEQ ID N°41), | GCCACUCCUGCCCCAUUUA | (SEQ ID N°69), |
| CCCCCGAGCAUGGAAGUAU | (SEQ ID N°42), | CUCUGGCAUUGCUGAUCAC |
| (SEQ ID N°43), | GCCUGUGAGUCUCUGGAUA | (SEQ ID N°44), |
| GCCACUCCUGCCCCAUUUA | (SEQ ID N°45), | GCCAGCAGGUCGGAAGUGA |
| (SEQ ID N°46), | AGUCUCUGGAUAUUCUCUC | (SEQ ID N°47), |
| UUUAUUGGCAGCCUGAUCG | (SEQ ID N°48), | UUGCUGAUCACUUCUGCGG |
| (SEQ ID N°49), | CUGGAUAUUCUCUCUGGCA | (SEQ ID N°50), |
| UCUGCCACUCCUGCCCCAU | (SEQ ID N-51), | AACCCCUUGGAGAGCCUCC |
| (SEQ ID N°52), | UGCCCAUGUCCCCAACCCC | (SEQ ID N°53), |
| AUAGAGAUAUCUGUUUGAA | (SEQ ID N°54), | CGAGCAUAGAGAUAUCUGU |
| (SEQ ID N°55), | CUUUGGGCAGCAUCAUAGU | (SEQ ID N°56), |
| AGACACCCCCAGGUCCUCU | (SEQ ID N°57), | CCUGGAACGGCUGAUGAGU |
| (SEQ ID N°58), | CCAAAUAAAUAGUAGUCUA | (SEQ ID N°59), |
| UCCAAUACAGUGCUGCUGU | (SEQ ID N°60), | CUCAGCUUUCUCGUUGGAC |
| (SEQ ID N°61), | CCAUUCCUCAGCUUUCUCG | (SEQ ID N°62), |
| CCGGCCCCAUUCCUCAGCU | (SEQ ID N°63), | CUUUGCCACUCCGGCCCCA |
| (SEQ ID N°64), ou | UCUGAAGCGGUCGGGGUCU. | (SEQ ID N°65) |

11. Composition ou méthode selon l'une quelconque des revendications précédentes, dans laquelle l'ARN interférant comprend une modification sur une partie base, sur une partie sucre ou sur une partie phosphate.

12. Composition ou méthode selon l'une quelconque des revendications 1, 3 et 5, dans laquelle la composition comprend en outre un second ARN interférant d'une longueur de 19 à 49 nucléotides, et comprenant une séquence nucléotidique sens, une séquence nucléotidique anti-sens et une région d'au moins 80% de complémentarité d'au moins 19 nucléotides entre les séquences sens et anti-sens ; dans laquelle la séquence anti-sens du second ARN interférant s'hybride, dans les conditions physiologiques, à une seconde partie de l'ARNm correspondant à la SEQ ID N°1 ou à la SEQ ID N°2 et la séquence anti-sens a une région d'au moins 80% de complémentarité contiguë d'au moins 19 nucléotides avec la seconde partie hybridante de l'ARNm correspondant à la SEQ ID N°1 ou à la SEQ ID N°2.

13. Composition selon les revendications 1, 3 et 5, dans laquelle la composition comprend une quantité efficace d'un mélange d'au moins quatre ARN interférants, chaque ARN interférant ayant une longueur de 19 à 49 nucléotides, et un vecteur acceptable au plan pharmaceutique, chaque ARN interférant comprenant : une séquence nucléotidique sens, une séquence nucléotidique anti-sens et une région d'au moins 80% de complémentarité contiguë d'au moins 19 nucléotides entre les séquences sens et anti-sens de chacun des quatre ARN interférants ; dans laquelle les séquences anti-sens du mélange s'hybrident, dans les conditions physiologiques, à une partie de l'ARNm correspondant à la SEQ ID N°2 commençant au nucléotide 175, 252, 276 et 325, respectivement, et ont une région d'au moins 80% de complémentarité contiguë d'au moins 19 nucléotides avec la partie hybridante de l'ARNm correspondant à la SEQ ID N°2 commençant au nucléotide 175, 252, 276 et 325, respectivement.

14. Composition selon la revendication 2 ou méthode selon la revendication 4, dans laquelle la composition comprend en outre un second ARN interférant d'une longueur de 19 à 49 nucléotides comprenant une seconde séquence nucléotidique ayant une région d'au moins 80% de complémentarité contiguë d'au moins 19 nucléotides avec une seconde partie hybridante de l'ARNm correspondant à la SEQ ID N°1 ou à la SEQ ID N°2.

15. Composition selon la revendication 2 ou méthode selon la revendication 4, dans laquelle la composition comprend une quantité efficace d'un mélange d'au moins quatre ARN interférants, chaque ARN interférant ayant une longueur de 19 à 49 nucléotides et le mélange comprenant : une première, une deuxième, une troisième et une quatrième séquence nucléotidique ayant une région d'au moins 80% de complémentarité contiguë d'au moins 19 nucléotides avec la partie hybridante de l'ARNm correspondant à la SEQ ID N°2 commençant au nucléotide 175, 252, 276 et 325, respectivement.

16. Composition ou méthode selon l'une quelconque des revendications précédentes, dans laquelle la composition est préparée pour administration par voie topique, intra-vitréale ou trans-sclérale.


### SEQUENCE LISTING

<110> Clark, Abbot F Wang, Wan-Heng MCNatt, Loretta
<120> RNAi INHIBITION OF SERUM AMYLOID A FOR TREATMENT OF GLAUCOMA
<130> 34576.44
<150> 60/638,706
   <151> 2004-12-23
<160> 69
<170> PatentIn version 3.3
<210> 1
   <211> 722
   <212> DNA
   <213> Homo sapiens
<400> 1
   aaggctcagt ataaatagca gccaccgctc cctggcaggc agggacccgc agctcagcta     60
   cagcacagat caggtgagga gcacaccaag gagtgatttt taaaacttac tctgttttct    120
   ctttcccaac aagattatca tttcctttaa aaaaaatagt tatcctgggg catacagcca    180
   taccattctg aaggtgtctt atctcctctg atctagagag caccatgaag cttctcacgg    240
   gcctggtttt ctgctccttg gtcctgggtg tcagcagccg aagcttcttt tcgttccttg    300
   gcgaggcttt tgatggggct cgggacatgt ggagagccta ctctgacatg agagaagcca    360
   attacatcgg ctcagacaaa tacttccatg ctcgggggaa ctatgatgct gccaaaaggg    420
   gacctggggg tgcctgggct gcagaagtga tcagcgatgc cagagagaat atccagagat    480
   tctttggcca tggtgcggag gactcgctgg ctgatcaggc tgccaatgaa tggggcagga    540
   gtggcaaaga ccccaatcac ttccgacctg ctggcctgcc tgagaaatac tgagcttcct    600
   cttcactctg ctctcaggag atctggctgt gaggccctca gggcagggat acaaagcggg    660
   gagagggtac acaatgggta tctaataaat acttaagagg tggaaaaaaa aaaaaaaaaa    720
   aa                                                                   722
<210> 2
   <211> 570
   <212> DNA
   <213> Homo sapiens
<400> 2
   agggacccgc agctcagcta cagcacagat cagcaccatg aagcttctca cgggcctggt 60
   tttctgctcc ttggtcctga gtgtcagcag ccgaagcttc ttttcgttcc ttggcgaggc 120
   ttttgatggg gctcgggaca tgtggagagc ctactctgac atgagagaag ccaattacat 180
   cggctcagac aaatacttcc atgctcgggg gaactatgat gctgccaaaa ggggacctgg 240
   gggtgcctgg gccgcagaag tgatcagcaa tgccagagag aatatccaga gactcacagg 300
   ccatggtgcg gaggactcgc tggccgatca ggctgccaat aaatggggca ggagtggcag 360
   agaccccaat cacttccgac ctgctggcct gcctgagaaa tactgagctt cctcttcact 420
   ctgctctcag gagacctggc tatgaggccc tcggggcagg gatacaaagt tagtgaggtc 480
   tatgtccaga gaagctgaga tatggcatat aataggcatc taataaatgc ttaagaggtc 540
   aaaaaaaaaa aaaaaaaaaa aaaaaaaaaa 570
<210> 3
   <211> 614
   <212> DNA
   <213> Homo sapiens
<400> 3
   tatagctcca cggccagaag ataccagcag ctctgccttt actgaaattt cagctggaga 60
   aaggtccaca gcacaatgag gcttttcaca ggcattgttt tctgctcctt ggtcatggga 120
   gtcaccagtg aaagctggcg ttcgtttttc aaggaggctc tccaaggggt tggggacatg 180
   ggcagagcct attgggacat aatgatatcc aatcaccaaa attcaaacag atatctctat 240
   gctcggggaa actatgatgc tgcccaaaga ggacctgggg gtgtctgggc tgctaaactc 300
   atcagccgtt ccagggtcta tcttcaggga ttaatagact actatttatt tggaaacagc 360
   agcactgtat tggaggactc gaagtccaac gagaaagctg aggaatgggg ccggagtggc 420
   aaagaccccg accgcttcag acctgacggc ctgcctaaga aatactgagc ttcctgctcc 480
   tctgctctca gggaaactgg gctgtgagcc acacacttct ccccccagac agggacacag 540
   ggtcactgag ctttgtgtcc ccaggaactg gtatagggca cctagaggtg ttcaataaat 600
   gtttgtcaaa ttga 614
<210> 4
   <211> 19
   <212> DNA
   <213> Artificial Sequence
<220>
   <223> targeting sequence
<400> 4
   tggggcagga gtggcaaag 19
<210> 5
   <211> 21
   <212> RNA
   <213> Artificial Sequence
<220>
   <223> sense strand
<400> 5
   uggggcagga guggcaaagu u 21
<210> 6
   <211> 21
   <212> RNA
   <213> Artificial sequence
<220>
   <223> antisense strand
<400> 6
   cuuugccacu ccugccccau u 21
<210> 7
   <211> 21
   <212> DNA
   <213> Artificial sequence
<220>
   <223> sense strand with 3' TT
<220>
   <221> misc_RNA
   <222> (1)..(19)
   <223> ribonucleotides
<220>
   <221> misc_feature
   <222> (20)..(21)
   <223> deoxyribonucleotides
<400> 7
   uggggcagga guggcaaagt t 21
<210> 8
   <211> 21
   <212> DNA
   <213> Artificial sequence
<220>
   <223> antisense strand with 3 'TT
<220>
   <221> misc_RNA
   <222> (1)..(19)
   <223> ribonucleotides
<220>
   <221> misc_feature
   <222> (20)..(21)
   <223> deoxyribonucleotides
<400> 8
   cuuugccacu ccugccccat t 21
<210> 9
   <211> 51
   <212> DNA
   <213> Artificial sequence
<220>
   <223> hairpin duplex with loop
<220>
   <221> misc_RNA
   <222> (1)..(21)
   <223> ribonucleotides
<220>
   <221> misc_feature
   <222> (22)..(30)
   <223> any, A, T/U, C, G
<220>
   <221> misc_feature
   <222> (31)..(51)
   <223> ribonucleotides
<400> 9
   uggggcagga guggcaaagu unnnnnnnnn cuuugccacu ccugccccau u 51
<210> 10
   <211> 19
   <212> DNA
   <213> Artificial sequence
<220>
   <223> targeting sequence
<400> 10
   agaccccaat cacttccga 19
<210> 11
   <211> 19
   <212> DNA
   <213> Artificial sequence
<220>
   <223> targeting sequence
<400> 11
   ttacatcggc tcagacaaa 19
<210> 12
   <211> 19
   <212> DNA
   <213> Artificial Sequence
<220>
   <223> targeting sequence
<400> 12
   gcttctcacg ggcctggtt 19
<210> 13
   <211> 19
   <212> DNA
   <213> Artificial Sequence
<220>
   <223> targeting sequence
<400> 13
   gccaattaca tcggctcag 19
<210> 14
   <211> 19
   <212> DNA
   <213> Artificial Sequence
<220>
   <223> targeting sequence
<400> 14
   atacttccat gctcggggg 19
<210> 15
   <211> 19
   <212> DNA
   <213> Artificial sequence
<220>
   <223> targeting sequence
<400> 15
   gtgatcagca atgccagag 19
<210> 16
   <211> 19
   <212> DNA
   <213> Artificial Sequence
<220>
   <223> targeting sequence
<400> 16
   tatccagaga ctcacaggc 19
<210> 17
   <211> 19
   <212> DNA
   <213> Artificial sequence
<220>
   <223> targeting sequence
<400> 17
   tcacttccga cctgctggc 19
<210> 18
   <211> 19
   <212> DNA
   <213> Artificial sequence
<220>
   <223> targeting sequence
<400> 18
   gagagaatat ccagagact 19
<210> 19
   <211> 19
   <212> DNA
   <213> Artificial sequence
<220>
   <223> targeting sequence
<400> 19
   cgatcaggct gccaataaa 19
<210> 20
   <211> 19
   <212> DNA
   <213> Artificial Sequence
<220>
   <223> targeting sequence
<400> 20
   ccgcagaagt gatcagcaa 19
<210> 21
   <211> 19
   <212> DNA
   <213> Artificial Sequence
<220>
   <223> targeting sequence
<400> 21
   tgccagagag aatatccag 19
<210> 22
   <211> 19
   <212> DNA
   <213> Artificial sequence
<220>
   <223> targeting sequence
<400> 22
   atggggcagg agtggcaga 19
<210> 23
   <211> 19
   <212> DNA
   <213> Artificial sequence
<220>
   <223> targeting sequence
<400> 23
   ggaggctctc caaggggtt 19
<210> 24
   <211> 19
   <212> DNA
   <213> Artificial sequence
<220>
   <223> targeting sequence
<400> 24
   ggggttgggg acatgggca 19
<210> 25
   <211> 19
   <212> DNA
   <213> Artificial sequence
<220>
   <223> targeting sequence
<400> 25
   ttcaaacaga tatctctat 19
<210> 26
   <211> 19
   <212> DNA
   <213> Artificial Sequence
<220>
   <223> targeting sequence
<400> 26
   acagatatct ctatgctcg 19
<210> 27
   <211> 19
   <212> DNA
   <213> Artificial Sequence
<220>
   <223> targeting sequence
<400> 27
   actatgatgc tgcccaaag 19
<210> 28
   <211> 19
   <212> DNA
   <213> Artificial Sequence
<220>
   <223> targeting sequence
<400> 28
   agaggacctg ggggtgtct 19
<210> 29
   <211> 19
   <212> DNA
   <213> Artificial Sequence
<220>
   <223> targeting sequence
<400> 29
   actcatcagc cgttccagg 19
<210> 30
   <211> 19
   <212> DNA
   <213> Artificial sequence
<220>
   <223> targeting sequence
<400> 30
   tagactacta tttatttgg 19
<210> 31
   <211> 19
   <212> DNA
   <213> Artificial Sequence
<220>
   <223> targeting sequence
<400> 31
   acagcagcac tgtattgga 19
<210> 32
   <211> 19
   <212> DNA
   <213> Artificial Sequence
<220>
   <223> targeting sequence
<400> 32
   gtccaacgag aaagctgag 19
<210> 33
   <211> 19
   <212> DNA
   <213> Artificial Sequence
<220>
   <223> targeting sequence
<400> 33
   cgagaaagct gaggaatgg 19
<210> 34
   <211> 19
   <212> DNA
   <213> Artificial sequence
<220>
   <223> targeting sequence
<400> 34
   agctgaggaa tggggccgg 19
<210> 35
   <211> 19
   <212> DNA
   <213> Artificial Sequence
<220>
   <223> targeting sequence
<400> 35
   tggggccgga gtggcaaag 19
<210> 36
   <211> 19
   <212> DNA
   <213> Artificial sequence
<220>
   <223> targeting sequence
<400> 36
   agaccccgac cgcttcaga 19
<210> 37
   <211> 19
   <212> RNA
   <213> Artificial Sequence
<220>
   <223> antisense strand
<400> 37
   cuuugccacu ccugcccca 19
<210> 38
   <211> 19
   <212> RNA
   <213> Artificial Sequence
<220>
   <223> antisense strand
<400> 38
   ucggaaguga uuggggucu 19
<210> 39
   <211> 19
   <212> RNA
   <213> Artificial Sequence
<220>
   <223> antisense strand
<400> 39
   uuugucugag ccgauguaa 19
<210> 40
   <211> 19
   <212> RNA
   <213> Artificial Sequence
<220>
   <223> antisense strand
<400> 40
   aaccaggccc gugagaagc 19
<210> 41
   <211> 19
   <212> RNA
   <213> Artificial Sequence
<220>
   <223> antisense strand
<400> 41
   cugagccgau guaauuggc 19
<210> 42
   <211> 19
   <212> RNA
   <213> Artificial Sequence
<220>
   <223> antisense strand
<400> 42
   cccccgagca uggaaguau 19
<210> 43
   <211> 19
   <212> RNA
   <213> Artificial Sequence
<220>
   <223> antisense strand
<400> 43
   cucuggcauu gcugaucac 19
<210> 44
   <211> 19
   <212> RNA
   <213> Artificial Sequence
<220>
   <223> antisense strand
<400> 44
   gccugugagu cucuggaua 19
<210> 45
   <211> 19
   <212> RNA
   <213> Artificial Sequence
<220>
   <223> antisense strand
<400> 45
   gccacuccug ccccauuua 19
<210> 46
   <211> 19
   <212> RNA
   <213> Artificial Sequence
<220>
   <223> antisense strand
<400> 46
   gccagcaggu cggaaguga 19
<210> 47
   <211> 19
   <212> RNA
   <213> Artificial Sequence
<220>
   <223> antisense strand
<400> 47
   agucucugga uauucucuc 19
<210> 48
   <211> 19
   <212> RNA
   <213> Artificial Sequence
<220>
   <223> antisense strand
<400> 48
   uuuauuggca gccugaucg 19
<210> 49
   <211> 19
   <212> RNA
   <213> Artificial Sequence
<220>
   <223> antisense strand
<400> 49
   uugcugauca cuucugcgg 19
<210> 50
   <211> 19
   <212> RNA
   <213> Artificial Sequence
<220>
   <223> antisense strand
<400> 50
   cuggauauuc ucucuggca 19
<210> 51
   <211> 19
   <212> RNA
   <213> Artificial Sequence
<220>
   <223> antisense strand
<400> 51
   ucugccacuc cugccccau 19
<210> 52
   <211> 19
   <212> RNA
   <213> Artificial Sequence
<220>
   <223> antisense strand
<400> 52
   aaccccuugg agagccucc 19
<210> 53
   <211> 19
   <212> RNA
   <213> Artificial Sequence
<220>
   <223> antisense strand
<400> 53
   ugcccauguc cccaacccc 19
<210> 54
   <211> 19
   <212> RNA
   <213> Artificial Sequence
<220>
   <223> antisense strand
<400> 54
   auagagauau cuguuugaa 19
<210> 55
   <211> 19
   <212> RNA
   <213> Artificial sequence
<220>
   <223> antisense strand
<400> 55
   cgagcauaga gauaucugu 19
<210> 56
   <211> 19
   <212> RNA
   <213> Artificial Sequence
<220>
   <223> antisense strand
<400> 56
   cuuugggcag caucauagu 19
<210> 57
   <211> 19
   <212> RNA
   <213> Artificial Sequence
<220>
   <223> antisense strand
<400> 57
   agacaccccc agguccucu 19
<210> 58
   <211> 19
   <212> RNA
   <213> Artificial Sequence
<220>
   <223> antisense strand
<400> 58
   ccuggaacgg cugaugagu 19
<210> 59
   <211> 19
   <212> RNA
   <213> Artificial Sequence
<220>
   <223> antisense strand
<400> 59
   ccaaauaaau aguagucua 19
<210> 60
   <211> 19
   <212> RNA
   <213> Artificial Sequence
<220>
   <223> antisense strand
<400> 60
   uccaauacag ugcugcugu 19
<210> 61
   <211> 19
   <212> RNA
   <213> Artificial sequence
<220>
   <223> antisense strand
<400> 61
   cucagcuuuc ucguuggac 19
<210> 62
   <211> 19
   <212> RNA
   <213> Artificial Sequence
<220>
   <223> antisense strand
<400> 62
   ccauuccuca gcuuucucg 19
<210> 63
   <211> 19
   <212> RNA
   <213> Artificial Sequence
<220>
   <223> antisense strand
<400> 63
   ccggccccau uccucagcu 19
<210> 64
   <211> 19
   <212> RNA
   <213> Artificial Sequence
<220>
   <223> antisense strand
<400> 64
   cuuugccacu ccggcccca 19
<210> 65
   <211> 19
   <212> RNA
   <213> Artificial Sequence
<220>
   <223> antisense strand
<400> 65
   ucugaagcgg ucggggucu 19
<210> 66
   <211> 19
   <212> DNA
   <213> Artificial sequence
<220>
   <223> targeting sequence
<400> 66
   tatccagaga ttctttggc 19
<210> 67
   <211> 19
   <212> DNA
   <213> Artificial Sequence
<220>
   <223> antisense strand
<400> 67
   tgaatggggc aggagtggc 19
<210> 68
   <211> 19
   <212> DNA
   <213> Artificial Sequence
<220>
   <223> antisense strand
<400> 68
   taaatggggc aggagtggc 19
<210> 69
   <211> 19
   <212> RNA
   <213> Artificial sequence
<220>
   <223> antisense strand
<400> 69
   gccacuccug ccccauuua 19